# EUROPEAN PATENT SPECIFICATION

(11) **EP 1 403 354 B1**
(45) Date of publication and mention of the grant of the patent: **09.05.2012**
(21) Application number: 03018120.0
(22) Date of filing: 08.08.2003
(51) Int. Cl.: C09K 11/06

(54) **1,3,6,8 tetrasubstituted pyrene compound, organic EL element using the same, and organic EL display using the same**
1,3,6,8 tetrasubstituierte Pyrene-Verbindung, derselbe verwendendes organisches EL-Element und derselbe verwendende organische EL-Anzeige
Composé de Pyrène 1,3,6,8 tetrasubstitué, l'élément organique EL et l'écran organique EL utilisant ce composé

(30) Priority: 28.08.2002 JP 2002248378
(43) Date of publication of application: 31.03.2004
(73) Proprietor: FUJIFILM Corporation, Minato-ku Tokyo (JP)
(72) Inventor: Sotoyama, Wataru, Kawasaki-shi, Kanagawa 211-8588 (JP); Sato, Hiroyuki, Kawasaki-shi, Kanagawa 211-8588 (JP); Matsuura, Azuma, Kawasaki-shi, Kanagawa 211-8588 (JP); Narusawa, Toshiaki, Kawasaki-shi, Kanagawa 211-8588 (JP)
(74) Representative: Körfer, Thomas

(56) References cited:
- US-A- 4 136 229
- SAITO G ET AL: JOURNAL OF MATERIALS CHEMISTRY, vol. 11, no. 3, 7 February 2001 (2001-02-07), pages 723-735, XP009021476
- DATABASE WPI Section Ch, Week 199436 Derwent Publications Ltd., London, GB; Class E14, AN 1994-290854 XP002262074 & JP 06 219973 A (RICOH KK), 9 August 1994 (1994-08-09)
- DATABASE WPI Section Ch, Week 199309 Derwent Publications Ltd., London, GB; Class E14, AN 1993-072121 XP002262075 & JP 05 021161 A (RICOH KK), 29 January 1993 (1993-01-29)

## Description

### CROSS-REFERENCE TO RELATED APPLICATIONS

This application is based upon and claims the benefit of priority from the prior Japanese Patent Application No. 2002-248378, filed in August 28, 2002.

### BACKGROUND OF THE INVENTION

### Field of the Invention

The present invention relates to a 1,3,6,8-tetrasubstituted pyrene compound suitable as a luminescent material in an organic electroluminescent (EL) element, an organic EL element comprising the 1,3,6,8-tetrasubstituted pyrene compound, and an organic EL display comprising the organic EL element.

### Description of the Related Art

Organic EL elements have features such as self-luminousness and rapid response, and receive high expectations to be applied to flat panel displays. A two-layer (multi-layer) organic EL element comprising an organic thin film having positive hole transport properties (positive hole transport layer) and an organic thin film having electron transport properties (electron transport layer) has been reported (C. W. Tang and S. A. VanSlyke, *Applied Physics Letters* vol.51, 913 (1987)), and, as a large area light-emitting element which emits light at a low voltage of 10V or less, organic EL elements have recently been attracting attention. Organic EL elements of the laminated type have the basic construction, anode/positive hole transport layer/light-emitting layer/electron transport layer/cathode, wherein the functions of the positive hole transport layer or electron transport layer may be added to that of the light-emitting layer as in the two-layer type.

It has been recently expected that organic EL elements will soon be applied to full color displays. In a full color display, it is necessary to have pixels emitting light of three primary colors, blue (B), green (G) and red (R) arranged on a display panel. There are three methods of doing this, i.e., (a) providing three types of organic EL elements, blue (B), green (G), and red (R); (b) separating the emitted light from an organic EL element emitting white light (which is mixed light of blue (B), green (G) and red (R)) by color filters; and (c) converting the light emission from an organic EL element that emits blue light into green (G) light and red (R) light by a color conversion layer using fluorescence.

On the other hand, in order to obtain an organic EL element having high light-emitting efficiency, it has been proposed to dope a small amount of molecules of pigment with high fluorescence as a guest material in a host material which is a main material so as to form a light-emitting layer having high light-emitting efficiency (C. W. Tang, S. A. VanSlyke, and C. H. Chen, Journal of Applied Physics vol.65, 3610 (1989)).

The related art does not provide an organic EL element with high light-emitting efficiency. Therefore, a request has been made on a novel and high-performance organic EL element.

### SUMMARY OF THE INVENTION

It is therefore an object of the present invention to provide a 1,3,6,8-tetrasubstituted pyrene compound suitable as a luminescent material in an organic EL element, an organic EL element having excellent light-emitting efficiency, emission luminance and color purity of green light, and a highly efficient organic EL display using the organic EL element.

1,3,6,8-tetrasubstituted pyrene compounds mentioned in claim 1 are suitable as a luminescent material for green light emission in an organic EL element, and an organic EL element and an organic EL display using the 1,3,6,8-tetrasubstituted pyrene compounds as a luminescent material have excellent light-emitting efficiency, emission luminance and color purity of green light, as well as higher efficiency and higher performance than those obtained in the related art.

The organic EL element of the present invention is defined according to claim 1.

The organic EL display of the present invention uses the organic EL element of the present invention. As the organic EL display of the present invention uses the organic EL element of the present invention, it has excellent light-emitting efficiency, emission luminance, and color purity of green light.

### BRIEF DESCRIPTION OF THE DRAWINGS

FIG. 1 is a schematic cross sectional view describing an example of the layer structure in an organic EL element according to the present invention.
FIG. 2 is a schematic view describing an example of the structure of an organic EL display (passive matrix panel) of a passive matrix method.
FIG. 3 is a schematic diagram describing an example of the circuit in an organic EL display (passive matrix panel) of the passive matrix method shown in FIG. 2.
FIG. 4 is a schematic view describing an example of the structure of an organic EL display (active matrix panel) of an active matrix method.
FIG. 5 is a schematic diagram describing an example of the circuit in an organic EL display (active matrix panel) of the active matrix method shown in FIG. 4.
FIG. 6 is a chart diagram describing an example of the IR spectrum of synthesized 1,3,6,8-tetrakis[N-(1-methylphenyl)-N-phenylamino]pyrene.
FIG. 7 is a chart diagram describing an example of the IR spectrum of synthesized 1,3,6,8-tetrakis[N-(1-naphthyl)-N-phenylamino]pyrene.

### DESCRIPTION OF THE PREFERRED EMBODIMENTS

### <1,3,6,8-tetrasubstituted pyrene compound>

The 1,3,6,8-tetrasubstituted pyrene compound of the present invention is represented by the following formula (1).

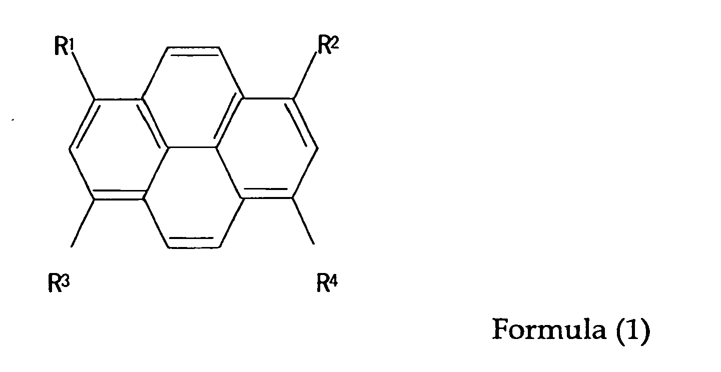

In the formula (1), R¹ to R⁴ may be the same or different, and each represent a group of the following formula (2).

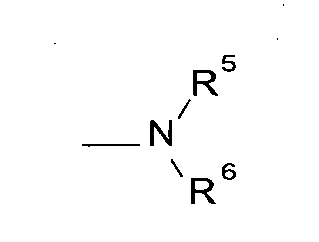

In the formula (2), R⁵ and R⁶ may be the same or different, and each represent an aryl group. R⁵ and R⁶ do not form a ring by bonding.

This may be further substituted by a substituent group. There is no particular limitation on the substituent group which further substitutes the above-mentioned aryl group. Examples of the substituent group can be selected from any of those known in the art.

An alkyl group can be selected according to the purpose. Examples of the alkyl group include a linear, branched or cyclic alkyl group having 1 to 10 carbon atoms. Specific examples of the alkyl group include methyl, ethyl, propyl, isopropyl, butyl, isobutyl, tertiary butyl, pentyl, isopentyl, hexyl, isohexyl, heptyl, isoheptyl, octyl, isooctyl, nonyl, isononyl, decyl, isodecyl, cyclopentyl, cyclobutyl, cyclopentyl, cyclohexyl, cycloheptyl, cyclooctyl, cyclononyl, cyclodecyl and the like.

An aryl group can be selected according to the purpose. Examples of the aryl group include a group having a monocyclic aromatic ring, a group where four or less of aromatic rings are bonded, and a group having five or less of fused aromatic rings and containing a total of 50 or less of carbon, oxygen, nitrogen and sulfur atoms.

There is no particular limitation on the monocyclic aromatic ring. The group having a monocyclic aromatic ring can be selected according to the purpose. Examples of the group having a monocyclic aromatic ring include phenyl, tolyl, xylyl, cumenyl, styryl, mesityl, cinnamyl, phenethyl, benzhydryl, and the like. These may be substituted by substituent groups.

There is no particular limitation on the group where four or less of aromatic rings are bonded. The group where four or less of aromatic rings are bonded can be selected according to the purpose. Examples of the group where four or less of aromatic rings are bonded include naphthyl, anthryl, phenanthryl, indenyl, azulenyl, benzanthracenyl, and the like. These may be substituted by substituent groups.

There is no particular limitation on the group having five or less of fused aromatic rings and containing a total of 50 or less of carbon, oxygen, nitrogen and sulfur atoms. The above-mentioned group can be selected according to the purpose. Examples of the above-mentioned group include pyrrolyl, furyl, thienyl, pyridyl, quinolyl, isoquinolyl, imidazoyl, pyridinyl, pyrrolopyridinyl, thiazoyl, pyrimidinyl, thiophenyl, indolyl, quinolinyl, pyrenyl, adenyl, and the like. These may be substituted by substituent groups.

It is preferred that R¹ to R⁴ in the formula (1) (represented by the formula (2)) are groups represented by one of the following formula (3), formula (4) and formula (5).

When R¹ to R⁴ in the formula (1) (each of which is represented by the formula (2)), are groups represented by the formula (3), the 1,3,6,8-tetrasubstituted pyrene compound is 1,3,6,8-tetrakis(N,N-diphenylamino) pyrene. When R¹ to R⁴ are groups represented by the formula (4), the 1,3,6,8-tetrasubstituted pyrene compound is 1,3,6,8-tetrakis [N-(1-naphthyl)-N-phenylamino] pyrene. When R¹ to R⁴ are groups represented by the formula (5), the 1,3,6,8-tetrasubstituted pyrene compound is 1,3,6,8-tetrakis[4,4'-bis(α,α-dimethylbenzyl)diphenylamino]pyrene.

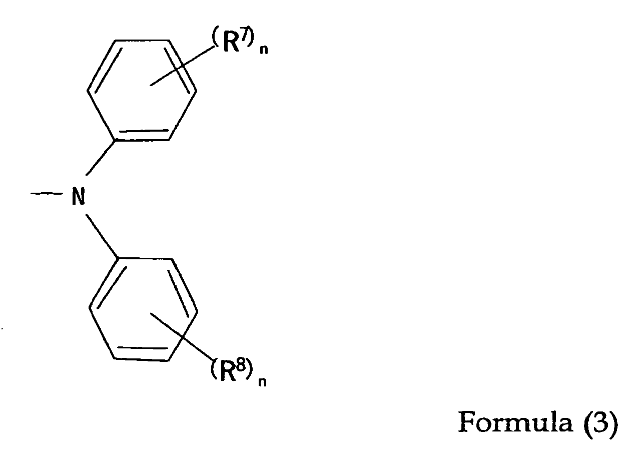

In the formula (3), R⁷ and R⁸ may be the same or different, and each represent at least one hydrogen atom, at least one alkyl group, or at least one aryl group. The alkyl group and aryl group may be any of those mentioned above. The letter "n" represents an integer of 1 or more.

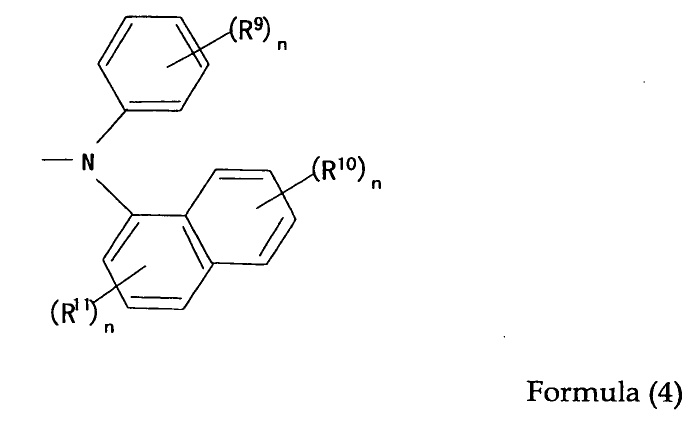

In the formula (4), R⁹, R¹⁰ and R¹¹ may be the same or different, and each represent at least one hydrogen atom, at least one alkyl group, or at least one aryl group. The alkyl group and aryl group may be any of those mentioned above. The letter "n" represents an integer of 1 or more.

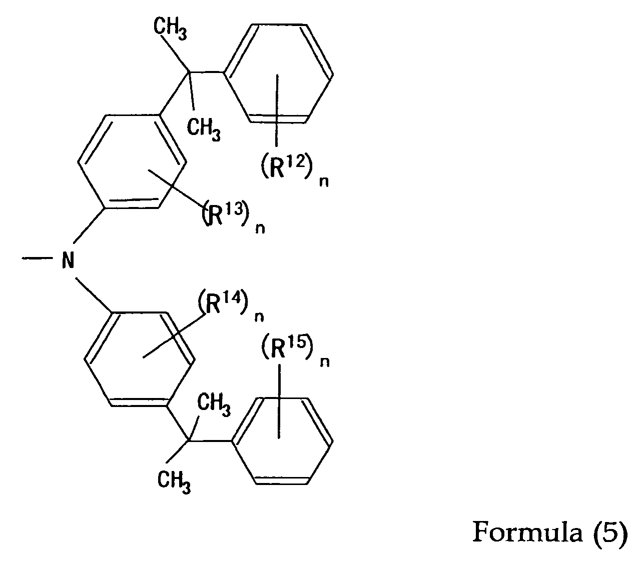

In the formula (5), R¹², R¹³, R¹⁴ and R¹⁵ may be the same or different, and each represent at least one hydrogen atom, at least one alkyl group, or at least one aryl group. The alkyl group and aryl group may be any of those mentioned above. The letter "n" represents an integer of 1 or more.

There is no particular limitation on a process for manufacturing the 1,3,6,8-tetrasubstituted pyrene compound of the present invention. The process may be suitably selected according to the purpose. An example of the process is given below. First, a 1,3,6,8-tetrahalogenated pyrene is synthesized by the reaction of pyrene and a halogen. The halogenation reaction may be performed by adding a simple halogen substance to the pyrene dissolved in a solvent, as described in *Annalen der Chemie,* vol. 531, page 81. Examples of the halogen include chlorine, bromine, iodine, and the like. These halogens are advantageous in the reaction of the next stage. Of these, chlorine and bromine are preferred as the halogenation reaction is easier. Next, the 1,3,6,8-tetrasubstituted pyrene compound of the present invention is obtained by heating the 1,3,6,8-tetrahalogenated pyrene and a secondary amine corresponding to the desired compound, and reacting them in the presence of a catalyst and a base. The catalyst may be copper or a copper compound, such as copper powder, cuprous chloride and copper sulfate, or a palladium compound, or the like, can be used. The base is sodium carbonate, potassium carbonate, sodium hydroxide, or a sodium alkoxide such as sodium-t-butoxide.

When 1,3,6,8-tetrakis[N-(1-methylphenyl)-N-phenylamino]pyrene is manufactured by the above general technique, 1,3,6,8-tetrabromopyrene is first obtained by the reaction of pyrene and bromine. Next, the 1,3,6,8-tetrabromopyrene is subjected to diarylamination according to a general process for synthesizing a triarylamine from an aryl halide as described in Tetrahedron Letters, Vol.39, page 2367 (1998). 4 equivalents of 3-methyl diphenylamine, 4 equivalents of sodium-t-butoxide, 0.1 equivalents of palladium acetate and 0.4 equivalents of tri(t-butyl) phosphine are added, and are reacted with the 1,3,6,8-tetrabromopyrene in o-xylene which serves as a solvent, at 130°C for 3 hours. The reaction liquid is washed several times with water after cooling, the o-xylene is distilled off, the remaining oil is washed with methanol, and the crude product is recrystallized from THF-methanol. The desired 1,3,6,8-tetrakis (N-(3-methylphenyl)-N-phenylamino)pyrene can then be obtained by purifying the crude product by vacuum sublimation.

The 1,3,6,8-tetrasubstituted pyrene compound of the present invention can be used in various fields, and is especially suitable as a luminescent material in organic EL elements. When the 1,3,6,8-tetrasubstituted pyrene compound of the present invention is used as a luminescent material in an organic EL element, green light emission is obtained.

### <Organic EL element>

The organic EL element of the present invention contains an organic thin film layer interposed between a positive electrode and a negative electrode. The organic thin film layer contains the 1,3,6,8-tetrasubstituted pyrene compound of the present invention. Namely, the organic EL element of the present invention contains the 1,3,6,8-tetrasubstituted pyrene compound represented by the aforementioned formula (1), as a luminescent material.

As mentioned above, R¹ to R⁴ in the formula (1) (groups represented by the formula (2)) are preferably represented by any one of the formula (3), the formula (4) and the formula (5).

In the present invention, the 1,3,6,8-tetrasubstituted pyrene compound is contained in the organic thin film layer as a luminescent material, and it may be contained in the light-emitting layer of the organic thin film layer, or in the light-emitting and electron transport layer, light-emitting and positive hole transport layer, or the like. When the 1,3,6,8-tetrasubstituted pyrene compound is contained in the light-emitting layer, this light-emitting layer may be formed as a film which contains only the 1,3,6,8-tetrasubstituted pyrene compound, or contains other materials in addition to the 1,3,6,8-tetrasubstituted pyrene compound.

In the present invention, it is preferred that the light-emitting layer in the organic thin film layer, light-emitting and electron transport layer, light-emitting and positive hole transport layer, and the like, comprises the 1,3,6,8-tetrasubstituted pyrene compound of the present invention as a guest material, and further comprises a host material in addition to the guest material having an emission wavelength near the optical absorption wavelength of the guest material. The host material is preferably contained in the luminescent layer, and may also be contained in the positive hole transport layer, electron transport layer, or the like.

In a case that the aforementioned guest material and host material are used in combination, when organic EL luminescence arises, the host material is excited first. As the emission wavelength of the host material and the absorption wavelength (330-500nm) of the guest material (1,3,6,8-tetrasubstituted pyrene compound) overlap, excitation energy is efficiently transferred from the host material to the guest material, the host material returns to the ground state without emitting light, and only the guest material which is in the excited state emits excitation energy as green light, therefore, light-emitting efficiency, emission luminance and color purity of green light are excellent.

In general, if only one kind of luminescent molecule is present or the molecules are contained at high concentration in a thin film, the luminescent molecules are so close to each other that they interact, and a so-called "concentration quenching" effect occurs wherein the light-emitting efficiency declines. However, when the guest material and host material are used together, the 1,3,6,8-tetrasubstituted pyrene compound which is the guest compound is dispersed at relatively low concentration in the host compound. Therefore, this "concentration quenching" effect is effectively suppressed and an excellent light-emitting efficiency is obtained. The use of the two materials in combination is therefore advantageous. Moreover, by using the guest material together with the host material in the light-emitting layer, as the host material generally has excellent film-forming properties, the combination has excellent film-forming properties while maintaining luminescent properties.

There is no particular limitation on the host material. The host material can be suitably selected according to the purpose. The emission wavelength is preferably close to the optical absorption wavelength of the guest material. Examples of the host material include the aromatic amine derivative represented by the following formula (6), the carbazole derivative represented by the formula (8), the oxine complex represented by the formula (10), 1,3,6,8-tetraphenylpyrene compound represented by the formula (21), 4,4'-(2,2'-diphenylvinyl)-1,1'-biphenyl (DPVBi) represented by the formula (14) (main emission wavelength = 470nm), p-sexiphenyl represented by the following formula (15) (main emission wavelength = 400nm) and 9,9'-bianthryl represented by the formula (16) (main emission wavelength = 460nm).

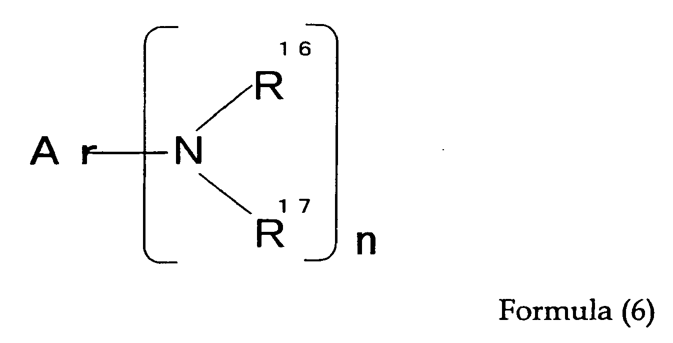

In the formula (6), "n" represents an integer of 2 or 3, Ar represents one of a divalent or trivalent aromatic group, and a divalent or trivalent heterocyclic aromatic group, R¹⁶ and R¹⁷ may be the same or different and represent a monovalent aromatic group or a heterocyclic aromatic group. There is no particular limitation on this aromatic group or heterocyclic aromatic group, and can be selected according to the purpose.

Of the aromatic amine derivatives represented by the formula (6),
N,N'-dinaphthyl-N,N'-diphenyl-[1,1'-biphenyl]-4,4'-diamine (NPD) which is represented by the following formula (7) (main emission wavelength = 430nm), and derivatives thereof, are preferred.

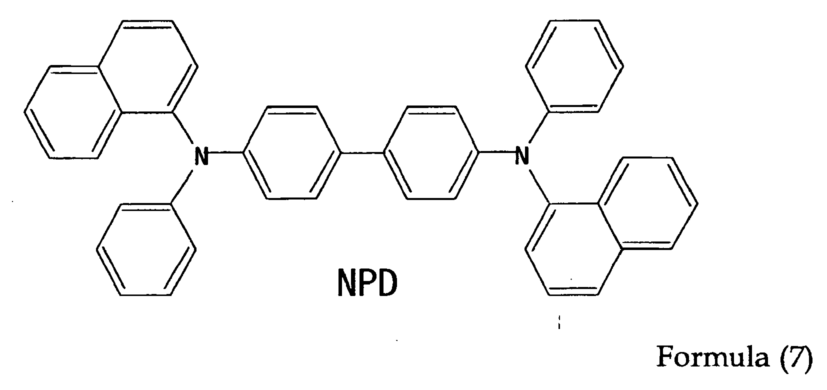

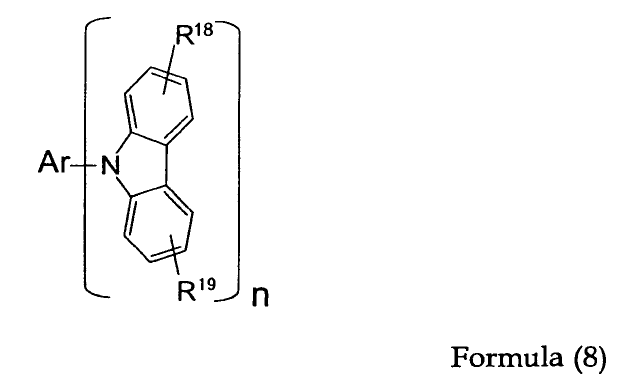

In the formula (8), Ar is a divalent or trivalent group which contains an aromatic ring shown below, or a divalent or trivalent group which contains a heterocyclic aromatic ring:

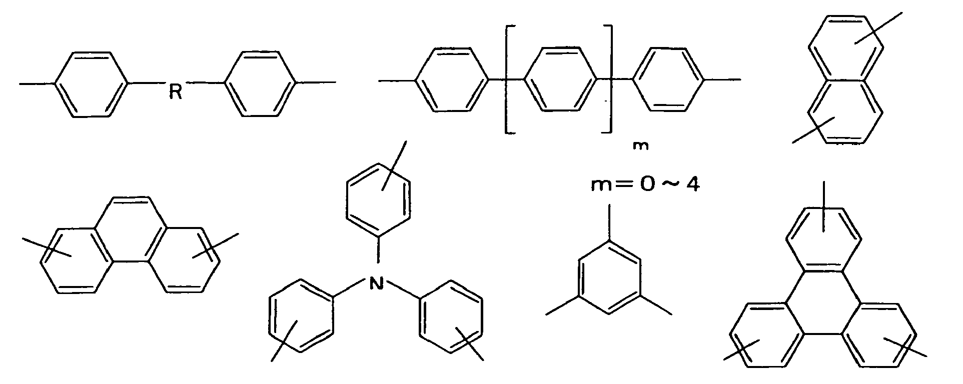

These may be substituted by a non-conjugated group. R is a linking group represented by, for example, the following formula:

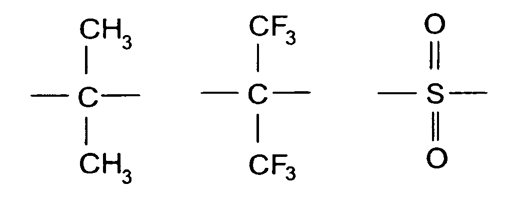

In the formula (8), R¹⁸ and R¹⁹ each represent a hydrogen atom, halogen atom, alkyl group, aralkyl group, alkenyl group, aryl group, cyano group, amino group, acyl group, alkoxycarbonyl group, carboxyl group, alkoxy group, alkylsulfonyl group, hydroxyl group, amide group, aryloxy group, aromatic hydrocarbon ring or aromatic heterocyclic group. These may be further substituted by substituents.

In the formula (8), "n" is preferably the integer of 2 or 3.

Of the aromatic amine derivative represented by the formula (8), a compound in which Ar is an aromatic group where two benzene rings are connected in a single bond, R¹⁸ and R¹⁹ are hydrogen atoms, and n=2, i.e., 4,4'-bis (9-carbazolyl)-biphenyl (CBP) represented by the following formula (9) (main emission wavelength = 380nm) and its derivatives are preferred in terms of particularly excellent light-emitting efficiency, emission luminance and color purity. 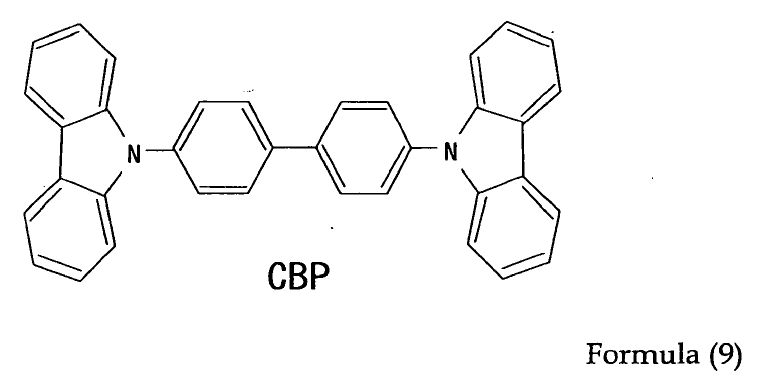 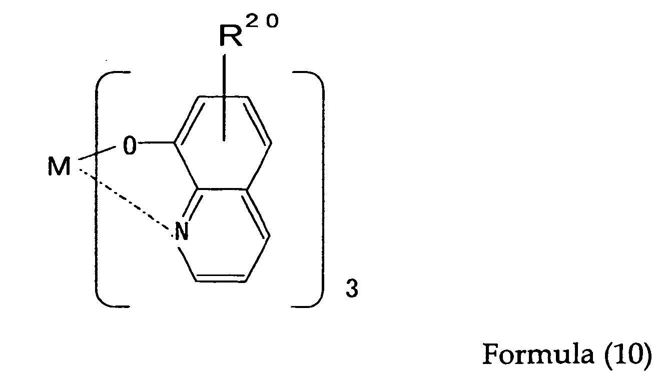 wherein "M" represents a metal atom, and R²⁰ represents a hydrogen atom, halogen atom, alkyl group, aralkyl group, alkenyl group, aryl group, cyano group, amino group, acyl group, alkoxycarbonyl group, carboxyl group, alkoxy group, alkylsulfonyl group, hydroxyl group, amide group, aryloxy group, aromatic hydrocarbon ring or aromatic heterocyclic group. These may be further substituted by substituents.

Of the oxine complexes represented by the formula (10), the aluminium quinoline complex (Alq) represented by the following formula (11) (main emission wavelength = 530nm) is preferred. 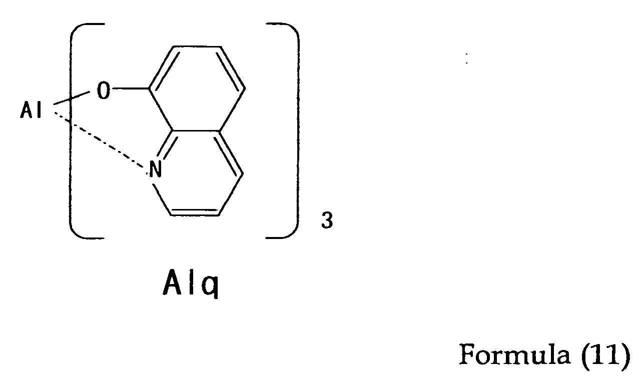 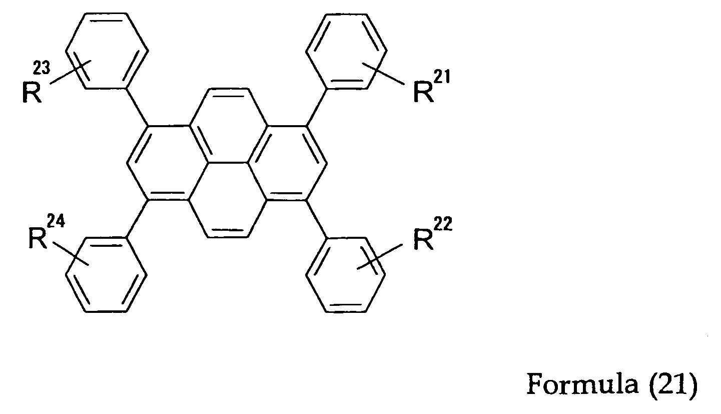 wherein R²¹ to R²⁴ each may be the same or different, and each represent a hydrogen atom or substituent group. This substituent may for example be an alkyl group, cycloalkyl group or aryl group, and these may be further substituted by substituents.

In the 1,3,6,8-tetraphenylpyrenes represented by the formula (21), when R²¹ to R²⁴ are hydrogen atoms, i.e., the 1,3,6,8-tetraphenylpyrene represented by the following formula (13) (main emission wavelength = 440nm) is preferred from the viewpoint of excellent light-emitting efficiency, emission luminance and color purity.

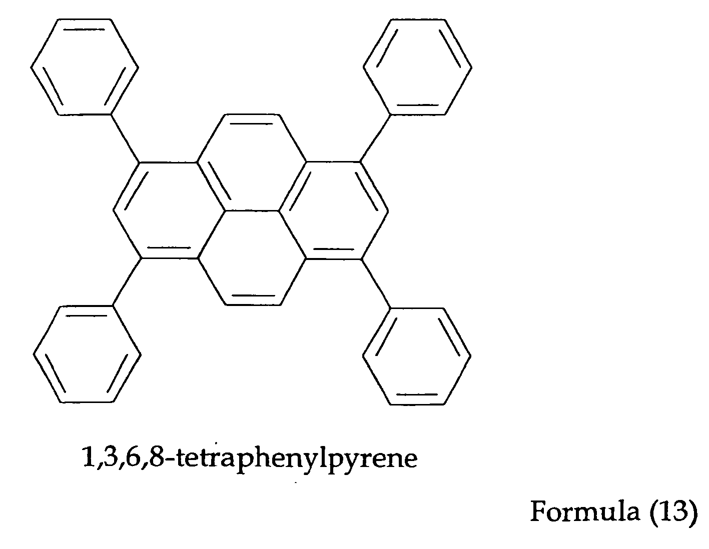

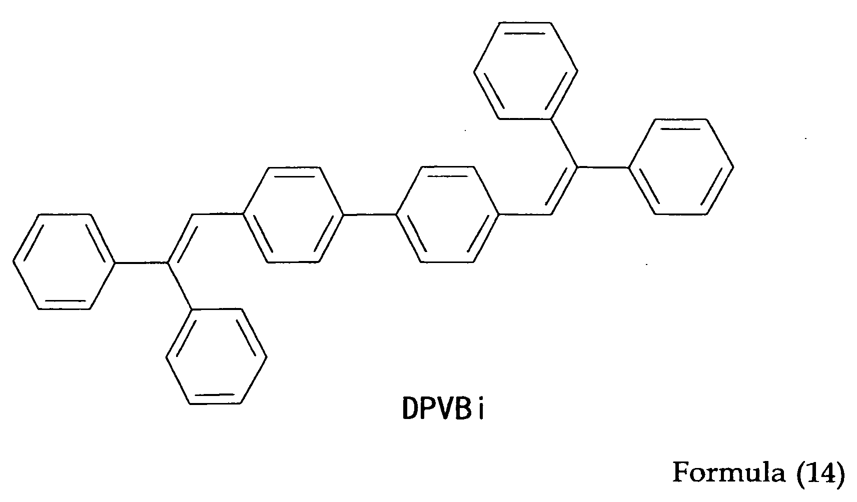

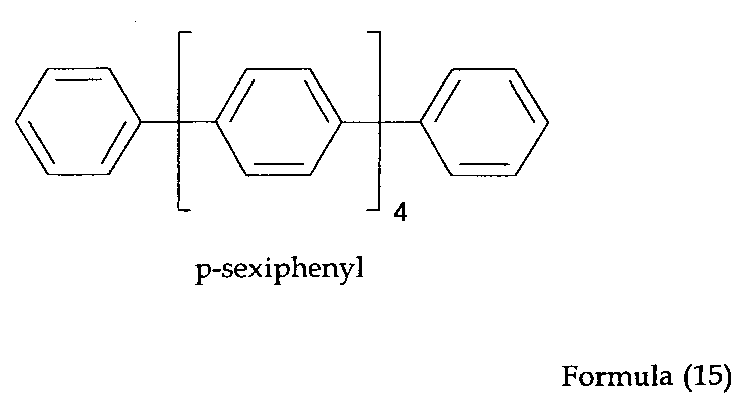

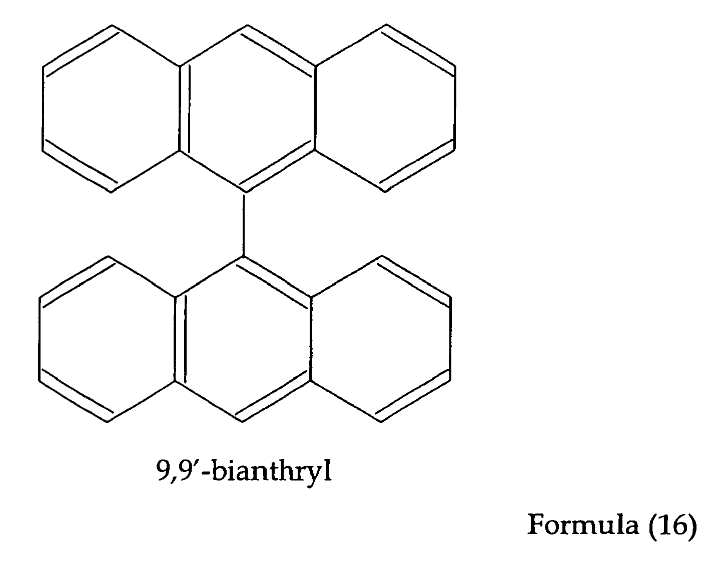

The amount of this 1,3,6,8-tetrasubstituted pyrene compound in a layer which contains the 1,3,6,8-tetrasubstituted pyrene compound represented by the formula (1), is preferably 0.1% by mass to 50 % by mass, and more preferably 0.5% by mass to 20 % by mass.

When the content is less than 0.1 % by mass, light-emitting efficiency, emission luminance and color purity may not be sufficient. When it is more than 50 % by mass, color purity may deteriorate. On the other hand, when the amount is within the preferred range, light-emitting efficiency, emission luminance and color purity are excellent.

Provided that the light-emitting layer in the organic EL element of the present invention may be injected with positive holes from the aforementioned positive electrode, a positive hole injection layer, the aforementioned positive hole transport layer or the like, and electrons from the aforementioned negative electrode, electron hole injection layer, the aforementioned electron transport layer or the like when an electric field is applied; provides a site for recombination of positive holes and electrons; and has a function to allow the luminescent 1,3,6,8-tetrasubstituted pyrene compound (luminescent material, luminous molecule) to emit green light using recombination energy generated by the recombination, the light-emitting layer may contain a luminescent material in addition to the 1,3,6,8-tetrasubstituted pyrene compound, as long as it does not interfere with the green light emission.

The aforementioned light-emitting layer can be formed according to any known methods. Examples of the methods include the vapor deposition method, wet film forming method, molecular beam epitaxy (MBE) method, cluster ion beam method, molecule laminating method, Langmuir-Brodgett (LB) method, printing method, transfer method, and the like.

Of these, vapor deposition is preferred from the viewpoint that an organic solvent is not used, and that there is no problem of waste fluid treatment, and the light-emitting layer can be manufactured at lower cost, with ease and efficiency, accordingly. When formed as a single layer structure, for example, formed as a positive hole transport and light-emitting and electron transport layer, the wet film forming method can also be preferred.

There is no particular limitation on the vapor deposition method. The vapor deposition can be suitably selected from known methods according to the purpose. Examples of the vapor deposition method include vacuum vapor deposition, resistance heating vapor deposition, chemical vapor deposition, physical vapor deposition, and the like. Examples of chemical vapor deposition include plasma CVD, laser CVD, heat CVD, gas source CVD, and the like. The light-emitting layer may be formed by, for example, subjecting the 1,3,6,8-tetrasubstituted pyrene compound to the vacuum vapor deposition. When containing the host material and the 1,3,6,8-tetrasubstituted pyrene compound, the light-emitting layer may be formed by simultaneously depositing the host material and the 1,3,6,8-tetrasubstituted pyrene compound by the vacuum vapor deposition. The light-emitting layer is more easily formed when not containing the host material, from the viewpoint that there is not need of co-deposition.

There is no particular limitation on the aforementioned wet film forming method. The wet film forming method can be suitably selected from known methods according to the purpose. Examples of the wet film forming method include the ink-jet method, spin coating method, kneader coat method, bar coating method, blade coating method, casting method, dip coating method, curtain coating method, and the like.

In the aforementioned wet film forming method, a solution, in which the material of the light-emitting layer is dissolved and dispersed with a resin component, may be used (applied or the like). Examples of the resin component include polyvinyl carbazole, polycarbonate, polyvinyl chloride, polystyrene, polymethyl methacrylate, polyester, polysulfone, polyphenylene oxide, polybutadiene, a hydrocarbon resin, a ketone resin, a phenoxy resin, polyamide, ethyl cellulose, vinyl acetate, an acrylonitrile butadiene styrene (ABS) resin, polyurethane, a melamine resin, an unsaturated polyester resin, an alkyde resin, an epoxy resin, a silicone resin, and the like.

When formed by the wet film forming method, the light-emitting layer can be formed by, for example, coating and drying a solution (coating solution) of the 1,3,6,8-tetrasubstituted pyrene compound and, if needed, a resin material dissolved in a solvent. When the light-emitting layer also contains a host material in addition to the 1,3,6,8-tetrasubstituted pyrene compound, the wet film forming method can be performed by coating and drying a solution (coating solution) of the 1,3,6,8-tetrasubstituted pyrene compound, the host material and the resin if necessary, dissolved in a solvent.

The thickness of the aforementioned light-emitting layer is preferably 1nm to 50nm, and more preferably 3nm to 20nm.

If the thickness of the light-emitting layer is 1nm to 50nm, light-emitting efficiency, emission luminance and color purity of the light emitted by the organic EL element are sufficient. If the thickness is 3nm to 20nm, these effects are more remarkable.

The organic EL element of the present invention comprises an organic thin film layer which contains a light-emitting layer interposed between an positive electrode and a negative electrode. The organic EL element of the present invention may include other layers such as a protective layer or the like, according to the purpose.

The organic thin film layer comprises the aforementioned light-emitting layer, and may also comprise a positive hole injection layer, a positive hole transport layer, a positive hole blocking layer or an electron transport layer, if necessary.

### - Positive electrode -

There is no particular limitation on the positive electrode. The positive electrode can be suitably chosen according to the purpose. Specifically, when the organic thin film layer comprises only the light-emitting layer, it is preferred to supply positive holes (carrier) to the light-emitting layer. When the organic thin film layer comprises a positive hole transport layer in addition to the light-emitting layer, it is preferred to supply positive holes (carrier) to the positive hole transport layer. When the organic thin film layer further comprises a positive hole injection layer, it is preferred to supply positive holes (carrier) to the positive hole injection layer.

There is no particular limitation on the material of the positive electrode. The material can be suitably selected according to the purpose. Examples of the material include metals, alloys, metal oxide, electrically conducting compounds, mixtures thereof, and the like. Of these, materials having a work function of 4eV or more are preferred.

Specific examples of the material of the positive electrode include electrically conducting metal oxides such as tin oxide, zinc oxide, indium oxide, indium tin oxide (ITO) or the like; metals such as gold, silver, chromium, nickel, or the like; mixtures or laminates of these metals and electrically conducting metal oxides; inorganic electrically conducting substances such as copper iodide and copper sulfide, organic electrically conducting materials such as polyaniline, polythiophene and polypyrrole, and laminates of these with ITO, and the like. These may be used singly, or in combination of two or more. Of these, the electrically conducting metal oxides are preferred, and ITO is particularly preferred from the viewpoints of productivity, high conductivity and transparency.

There is no particular limitation on the thickness of the positive electrode. The thickness can be selected according to the material or the like. The thickness is preferably 1nm to 5,000nm, and more preferably 20nm to 200nm.

The positive electrode is typically formed on a substrate such as glass, soda lime glass, non-alkali glass, a transparent resin, or the like.

When using the above-mentioned glass as the substrate, non-alkali glass or soda lime glass with a barrier layer of silica or the like, are preferred from the viewpoint that they lessen transport of ions from the glass.

There is no particular limitation on the thickness of the substrate as long as it is sufficient to maintain mechanical strength. When using glass as the substrate, however, it is typically 0.2mm or more, and preferably 0.7mm or more.

The positive electrode can be conveniently formed by known methods such as vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method and the method of applying a dispersion of ITO by a chemical reaction method (sol gel process, or the like).

By washing the positive electrode and performing other treatment, the driving voltage of the organic EL element can be reduced, and the light-emitting efficiency can also be increased. When the material of the positive electrode is ITO, examples of the other treatment include UV ozonization and plasma processing, and the like.

### - Negative electrode -

There is no particular limitation on the negative electrode. The negative electrode can be suitably selected according to the purpose. It is preferred that the anode supplies positive holes (carriers) to the organic thin film layer, specifically, to a light-emitting layer when the organic thin film layer comprises only the light-emitting layer or when the organic thin film layer comprises an electron transport layer in addition to the light-emitting layer, it is preferred to supply electrons to this electron transport layer. When an electron hole injection layer is interposed between the organic thin film layer and the negative electrode, it is preferred to supply electrons to the electron hole injection layer.

There is no particular limitation on the material of the negative electrode. The material can be suitably selected according to adhesion properties with the layers or molecules adjoining this negative electrode, such as the electron transport layer and light-emitting layer, and according to ionization potential, and stability. Examples of the material include metals, alloys, metal oxides, electrically conducting compounds, mixtures thereof, and the like.

Examples of the material of the negative electrode include alkali metals (for example, Li, Na, K, Cs), alkaline earth metals (e.g., Mg, Ca), gold, silver, lead, aluminum, sodium-potassium alloys or mixtures thereof, lithium-aluminium alloys or mixtures thereof, magnesium-silver alloys or mixtures thereof, rare earth metals such as indium and ytterbium, and alloys thereof.

These may be used singly, or in combination of two or more. Of these, materials having a work function of 4eV or less are preferred. Aluminum, lithium-aluminium alloys or mixtures thereof, or magnesium-silver alloys or mixtures thereof, are more preferred.

There is no particular limitation on the thickness of the negative electrode. The thickness can be chosen according to the material of the negative electrode. The thickness is preferably 1nm to 10,000nm, and more preferably 20nm to 200nm.

The negative electrode can be conveniently formed by known methods such as the vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method, or the like.

When two or more of these are used together as the material of the negative electrode, two or more materials may be vapor-deposited simultaneously to form an alloy electrode, or a pre-prepared alloy may be made to vapor-deposit so as to form an alloy electrode.

The resistances of the positive electrode and negative electrode are preferably low, and it is preferred that they are not more than several hundred ohms per square.

### - Positive hole injection layer -

There is no particular restriction on the positive hole injection layer. The positive hole injection layer can be chosen according to the purpose. It is preferred that it has the function of, for example, injecting positive holes from the positive electrode when an electric field is applied.

There is no particular limitation on the material of the positive hole injection layer which can be conveniently chosen according to the purpose, for example, a starburst amine (4, 4', 4"-tris[3-methylphenyl(phenyl) amino] triphenylamine:m-MTDATA) represented by the following formula, copper phthalocyanine or polyaniline.

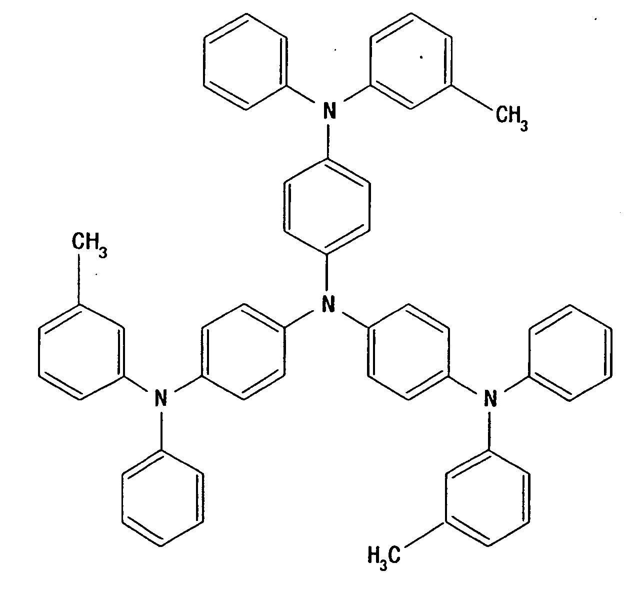

There is no particular limitation on the thickness of the positive hole injection layer which can be chosen according to the purpose. The thickness is preferably 1nm to 100nm, and more preferably 5nm to 50nm.

The positive hole injection layer can be conveniently formed by known the methods such as the vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method, or the like.

### - Positive hole transport layer -

There is no particular limitation on the positive hole transport layer. The positive hole transport layer can be chosen according to the purpose. Examples of the positive hole transport layer include a layer having the function to convey positive holes from the positive electrode when an electric field is applied, is preferred.

There is no particular limitation on the material of the positive hole transport layer. The material can conveniently be chosen according to the purpose. Examples of the material include aromatic amine compounds, carbazole, imidazole, triazole, oxazole, oxadiazole, polyarylalkane, pyrrazoline, pyrrazolone, phenylene diamine, arylamine, amino-substituted chalcone, styryl anthracene, fluorenone, hydrazone, stilbene, silazane, styryl amine, aromatic dimethylidene compounds, porphyrine compounds, electrically conducting oligomers and polymers such as polisilane compounds, poly(N-vinyl carbazole), aniline copolymers, thiophene oligomers and polymers, polythiophene and carbon film. If the materials of the positive hole transport layers is mixed with the materials of the light-emitting layer to form a film, a positive hole transport and light-emitting layer can be formed.

These may be used singly, or two or more may be used in combination. Of these, aromatic amine compounds are preferred. Specific examples of the aromatic amine compounds include TPD (N, N'-diphenyl-N, N'(-bis (3-methylphenyl)-[1,1'-biphenyl]-4, 4'-diamine) represented by the following formula, and NPD (N, N'-dinaphthyl-N, N'-diphenyl-[1,1'-biphenyl]-4,4'-diamine) represented by the following formula, are more preferred.

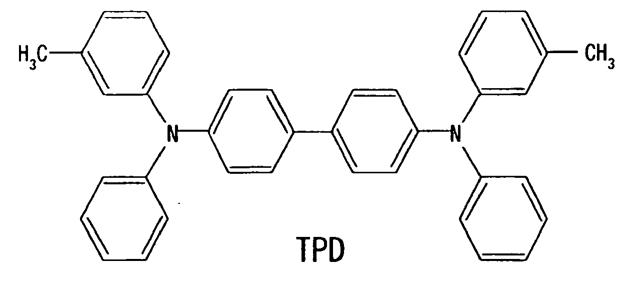

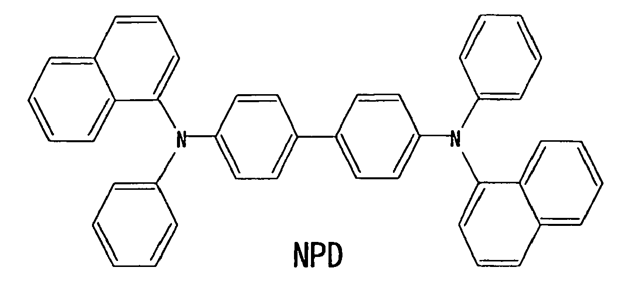

There is no particular limitation on the thickness of the positive hole transport layer. The thickness of the positive hole transport layer may be chosen according to the purpose. The thickness is typically in the range of 1nm to 500nm, and more preferably 10nm to 100nm.

The positive hole transport layer can be suitably formed by known methods such as the vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method, or the like.

### - Positive hole blocking layer -

There is no particular limitation on the positive hole blocking layer. The positive hole blocking layer may be chosen according to the purpose. The positive hole blocking layer preferably has the function of a barrier to positive holes injected from the positive electrode.

There is no particular limitation on the material of the positive hole blocking layer. The material can be suitably chosen according to the purpose.

If the aforementioned organic EL element comprises a positive hole blocking layer, positive holes conveyed from the positive electrode side are blocked by this positive hole blocking layer, and electrons conveyed from the negative electrode are transmitted through this positive hole blocking layer to reach the aforementioned light-emitting layer. Hence, recombination of electrons and positive holes occurs efficiently in this light-emitting layer, and recombination of positive holes and electrons in organic thin film layers other than this light-emitting layer can be prevented. Thus, the luminescence from the 1,3,6,8-tetrasubstituted pyrene compound which is the target luminescent material is obtained efficiently. This is advantageous in respect of color purity.

The positive hole blocking layer is preferably disposed between the light-emitting layer and the electron transport layer.

There is no particular limitation on the thickness of the positive hole blocking layer. The thickness can be suitably chosen according to the purpose. The thickness is typically about 1nm to 500nm, and more preferably 10nm to 50nm.

The positive hole blocking layer may be a single layer structure, or may be a laminated structure.

The positive hole blocking layer can be conveniently formed by known methods such as the vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method, or the like.

### - Electron transport layer -

There is no particular limitation on the electron transport layer. The electron transport layer can be suitably be chosen according to the purpose. Examples of the electron transport layer include a layer having one of the function to convey electrons from the negative electrode, and the function to act as a barrier to positive holes injected from the positive electrode, is preferred.

There is no particular limitation on materials of the electron transport layer. The materials can be selected according to the purpose. Examples of the materials include a quinoline derivative such as the aforementioned aluminum quinoline complex (Alq) or the like, an oxadiazole derivative, a triazole derivative, a phenanthroline derivative, a perylene derivative, a pyridine derivative, a pyrimidine derivative, a quinoxaline derivative, a diphenylquinone derivative, a nitro-substituted fluorene derivative, and the like. If an electron transport layer material is mixed with the light-emitting layer material to form a film, an electron transport and light-emitting layer can be formed. If a positive hole transport layer material is additionally mixed to form a film, an electron transport and positive hole transport and light-emitting layer can be formed. In this case, a polymer such as polyvinyl carbazole or polycarbonate can be used.

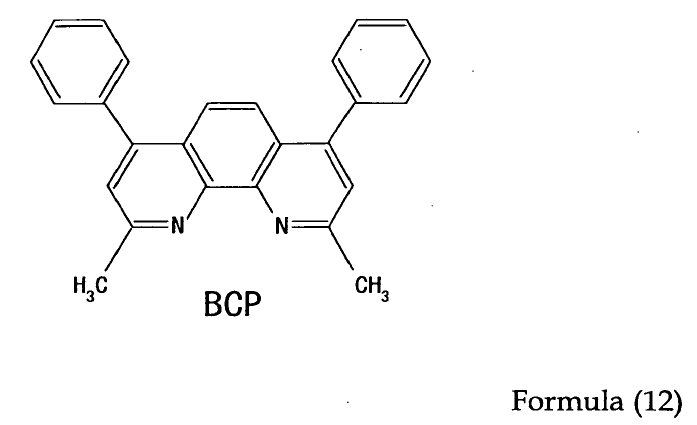

There is no particular limitation on the thickness of the electron transport layer. The thickness can be suitably chosen according to the purpose. The thickness is typically about 1nm to 500nm, and preferably 10nm to 50nm.

The electron transport layer may be a single layer structure, or may be a laminated layer structure.

In this case, it is preferred that an electron transport material used for the electron tranport layer adjacent to the light-emitting layer has an optical absorption edge at a shorter wavelength than that of the 1,3,6,8-tetrasubstituted pyrene compound so that it limits the luminescence region in the organic EL element to the light-emitting layer and prevents unwanted luminescence from the electron transport layer. Examples of the electron transport materials having an optical absorption edge at a shorter wavelength than that of the 1,3,6,8-tetrasubstituted pyrene compound, include phenanthroline derivatives, oxadiazole derivatives and triazole derivatives. The compounds shown below are preferable examples.

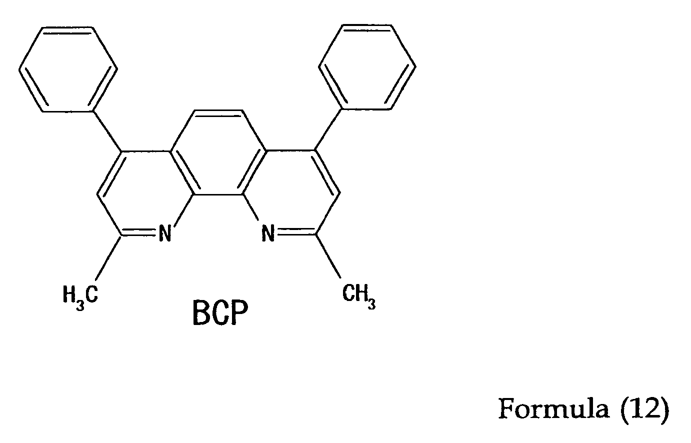

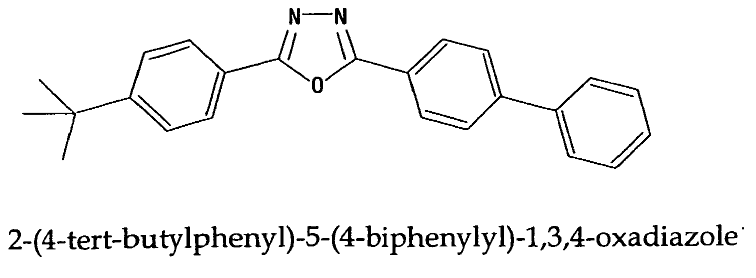

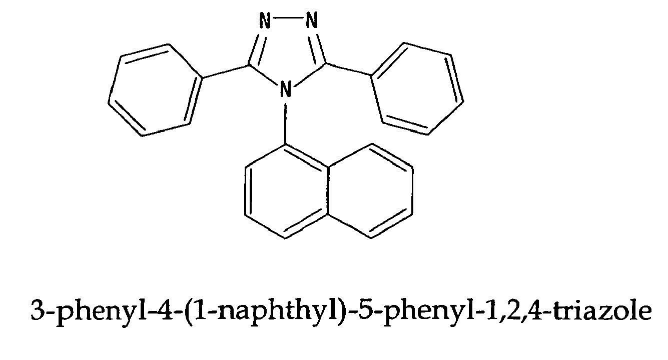

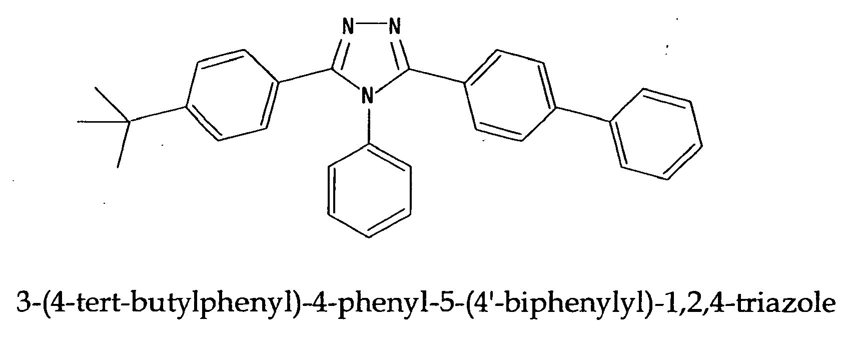

The electron transport layer can be conveniently formed by known methods such as the vapor deposition method, wet film forming method, electron beam method, sputtering method, reactive sputtering method, Molecular beam epitaxy (MBE) method, cluster ion beam method, ion plating method, plasma polymerization method (high frequency excitation ion plating method), molecule laminating method, Langmuir-brodgett (LB) method, printing method, transfer method, or the like.

### - Other layers -

The organic EL element of the present invention may have other layers. The other layers can be suitably chosen according to the purpose. Examples of the other layers include a protective layer, and the like.

There is no particular limitation on the aforementioned protective layer. The protective layer may be suitably chosen according to the purpose. Examples of the protective layers include a layer which can prevent molecules or substances that promote deterioration of the organic EL element, such as moisture and oxygen, from penetrating the organic EL element, is preferred.

Examples of the material of the aforementioned protective layer include metals such as In, Sn, Pb, Au, Cu, Ag, Al, Ti, and Ni, metal oxides such as MgO, SiO and SiO₂, Al₂O₃, GeO, NiO, CaO, BaO, Fe₂O₃, Y₂O₃ and TiO₂, nitrides such as SiN and SiNₓO_{y}, metal fluorides such as MgF₂, LiF, AlF₃, CaF₂, polyethylene, polypropylene, polymethyl methacrylate, polyimide, polyurea, polytetrafluoroethylene, polychlorotrifluoroethylene, polydichlorodifluoroethylene, a copolymer of chlorotrifluoroethylene and dichlorodifluoroethylene, a copolymer obtained by copolymerizing a monomer mixture comprising tetrafluoroethylene and at least one comonomer, a fluorine-containing copolymer having a ring structure in a main chain of the copolymer, a water-absorbing substance having a water absorption rate of 1% or more, and a dampproof substance having a water absorption rate of 0.1% or less.

The protective layer can be suitably formed by known methods such as the vapor deposition method, wet film forming method, sputtering method, reactive sputtering method, Molecular beam epitaxy (MBE) method, cluster ion beam method, ion plating method, plasma polymerization method (high frequency excitation ion plating method), printing method and transfer method.

There is no particular limitation on the structure of the organic EL element of the present invention. The structure may be selected according to the purpose. Examples of the structures include the following (1) to (13):
(1) Positive electrode/positive hole injection layer/positive hole transport layer/light-emitting layer/electron transport layer/electron hole injection layer/negative electrode,
(2) Positive electrode/positive hole injection layer/positive hole transport layer/light-emitting layer/electron transport layer/negative electrode,
(3) Positive electrode/positive hole transport layer/light-emitting layer/electron transport layer/electron hole injection layer/negative electrode,
(4) Positive electrode/positive hole transport layer/ light-emitting layer/electron transport layer/negative electrode,
(5) Positive electrode/positive hole injection layer/positive hole transport layer/light-emitting and electron transport layer/electron hole injection layer/negative electrode
(6) Positive electrode/positive hole injection layer/positive hole transport layer/light-emitting and electron transport layer/negative electrode,
(7) Positive electrode/positive hole transport layer/light-emitting and electron transport layer/electron hole injection layer/negative electrode,
(8) Positive electrode/positive hole transport layer/light-emitting and electron transport layer/negative electrode,
(9) Positive electrode/positive hole injection layer/positive hole transport and light-emitting layer/electron transport layer/electron hole injection layer/negative electrode
(10) Positive electrode/positive hole injection layer/positive hole transport and light-emitting layer/electron transport layer/negative electrode,
(11) Positive electrode/positive hole transport and light-emitting layer/electron transport layer/electron hole injection layer/negative electrode,
(12) Positive electrode/positive hole transport and light-emitting layer/electron transport layer/negative electrode,
(13) Positive electrode/positive hole transport and light-emitting and electron transport layer/negative electrode, and the like.

When the organic EL element has a positive hole blocking layer, layer configurations in which the positive hole blocking layer is interposed between the light-emitting layer and electron transport layer in the configuration (1) to (13) presented above may also be suitable.

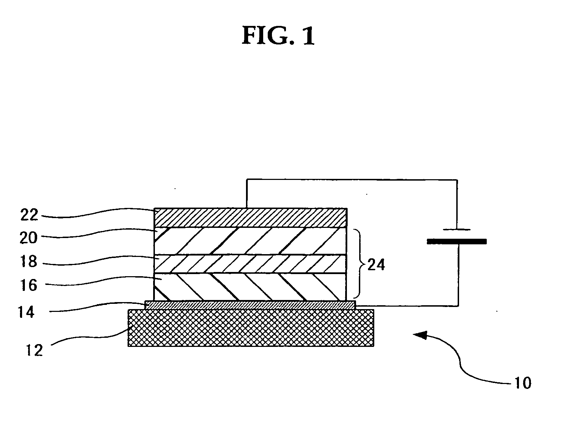

Of these layer structures, the aspect (4), positive electrode/positive hole transport layer/light-emitting layer/electron transport layer/negative electrode, is shown in FIG. 1. An organic EL element 10 has a layer structure comprising an positive electrode 14 (for example, ITO electrode) formed on a glass substrate 12, a positive hole transport layer 16, a light-emitting layer 18, an electron transport layer 20, and a negative electrode 22 (for example, an Al-Li electrode), each of which is disposed in this order. The positive electrode 14 (for example, an ITO electrode) and the negative electrode 22 (for example, an Al-Li electrode) are interconnected through the power supply. An organic thin film layer 24 which emits green light is formed by the positive hole transport layer 16, light-emitting layer 18 and electron transport layer 20.

The luminescence peak wavelength of the organic EL element of the present invention is preferably 490nm to 540nm.

From the viewpoint of light-emitting efficiency of the organic EL element of the present invention, it is preferred that it emits green light at a voltage of 10V or less, more preferred that it emits green light at a voltage of 7V or less, and still more preferred that it emits green light at a voltage of 5V or less.

It is preferred that, at an applied voltage of 10V, the emission luminance of the organic EL element of the present invention is 100 cd/m² or more, more preferred that it is 500 cd/m² or more, and still more preferred that it is 1,000 cd/m² or more.

The organic EL element of the present invention is especially useful in various fields such as computers, vehicle-mounted display devices, field-ready display devices, home apparatuses, industrial apparatuses, household electric appliances, traffic display devices, clock display devices, calendar display units, luminescent screens and audio equipment, and is particularly suitable for the organic EL display of the present invention, as described below.

### <Organic EL display>

There is no particular limitation on the organic EL display of the present invention. The organic EL display of the present invention can be chosen from known structure, except that it uses the organic EL element of the present invention.

The organic EL display may be a green monochrome, a multi-color, or a full color type.

The organic EL display may be made a full color type as disclosed in *Monthly Display* (published by Techno Times Co., Ltd. of Japan), September 2000, pages 33-37, i.e., (a) the three color light emitting method where three types of organic EL elements which, respectively, emit light corresponding to the three primary colors (blue (B), green (G), red (R)) are disposed on a substrate; (b) the white method where white light from an organic EL element for white light emission is divided into the three primary colors via color filters; (c) and the color conversion method wherein blue light emitted by an organic EL element which emits blue light is converted into red (R) and green (G) via a fluorescent pigment layer. In the present invention, as the organic EL element of the invention emits green light, the three color light emitting method and color conversion method can be used, the three color light emitting method being particularly suitable.

To manufacture an organic EL display of the full color type by the three color light emitting method, an organic EL element for red light emission and an organic EL element for blue light emission are required in addition to the organic EL element of the present invention for green light emission.

There is no particular limitation on the organic EL element for red light emission. The organic EL element for red light emission can be selected from among known in the art. A typical laminar construction is ITO (positive electrode)/NPD/DCJTB 1% aluminium quinoline complex (Alq) as shown below/Alq/Al-Li (negative electrode). DCJTB is 4-dicyanomethylene-6-cp-julolidinostyryl-2-tert-butyl-4H-pyran. Alq is as shown previously.

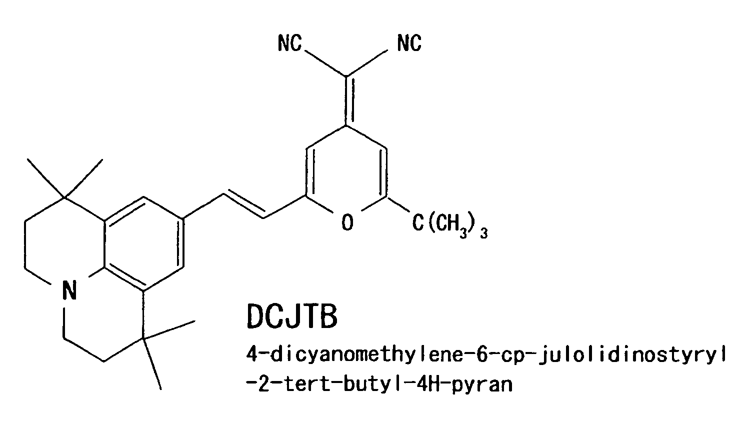

There is no particular limitation on the organic EL element used for blue light emission. The organic EL element can be selected from among known in the art. Examples of a suitable laminar structure include ITO (positive electrode)/NPD/DPVBi/Alq/Al-Li (negative electrode).

There is no particular limitation on embodiments of the organic EL display. The embodiments can be chosen according to the purpose. Suitable examples of the embodiments include the passive matrix panel and active matrix panel disclosed in Nikkei Electronics (published by Nikkei Business Publications Inc. of Japan), No. 765, March 13, 2000, pages 55-62.

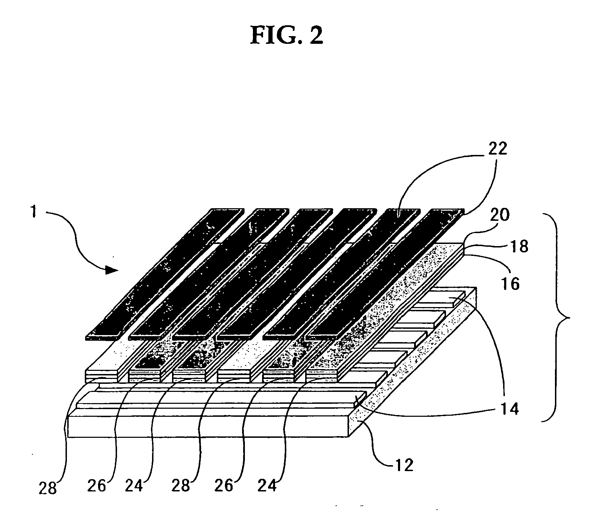

The aforementioned passive matrix panel for example has belt-like positive electrodes 14 (for example, ITO electrodes) arranged parallel to each other on a glass substrate 12 as shown in FIG. 2. On the anodes 14, belt-like organic thin film layers 24 for green light emission, organic thin film layers 26 for blue light emission and organic thin film layers 28 for red light emission are arranged sequentially in parallel and substantially perpendicular to the positive electrodes 14. Each of the organic thin film layers has negative electrodes 22 of the same shape thereon.

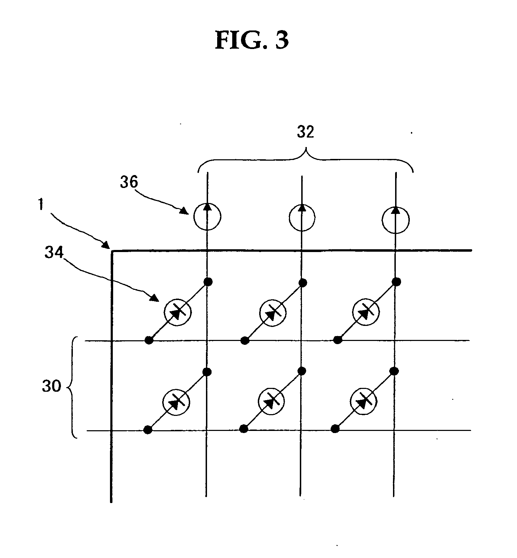

In the aforementioned passive matrix panel, positive electrode lines 30 comprising plural positive electrodes 14, and negative electrode lines 32 comprising plural negative electrodes 22, for example intersect substantially at right angles to form a circuit, as shown in FIG. 3. Each of the organic thin film layers 24, 26, and 28 for green light emission, blue light emission and red light emission situated at each intersection point functions as a pixel, there being plural organic EL elements 34 corresponding to each pixel. In this passive matrix panel, when a current is applied by a constant current supply 36 to one of the positive electrodes 14 in the positive electrode lines 30, and one of the negative electrodes 22 in the negative electrode lines 32, a current will be applied to the organic EL thin film layer situated at the intersection, and the organic EL thin film layer at this position will emit light. By controlling the light emission of each pixel unit, a full color picture can easily be formed.

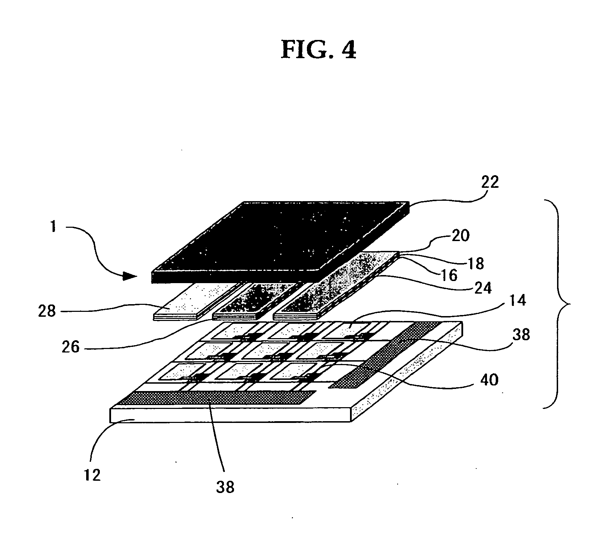

In the active matrix panel, for example, scanning lines, data lines and current supply lines are arranged in a grid pattern on a glass substrate 12, as shown in FIG. 4. A TFT circuit 40 connected to the scanning lines and the like forming the grid pattern is disposed in each grid, and an positive electrode 14 (for example, an ITO electrode) disposed in each grid can be driven by the TFT circuit 40. On the anodes 14, a belt-like organic thin film layer 24 for green light emission, organic thin film layer 26 for blue light emission and organic thin film layer 28 for red light emission, are arranged sequentially and in parallel to each other. A negative electrode 22 is also arranged so as to cover each of the organic thin film layer 24 for green light emission, organic thin film layer 26 for blue light emission and organic thin film layer 28 for red light emission. The organic thin film layer 24 for green light emission, organic thin film layer 26 for blue light emission and organic thin film layer 28 for red light emission respectively comprise a positive hole transport layer 16, light-emitting layer 18 and electron transport layer 20.

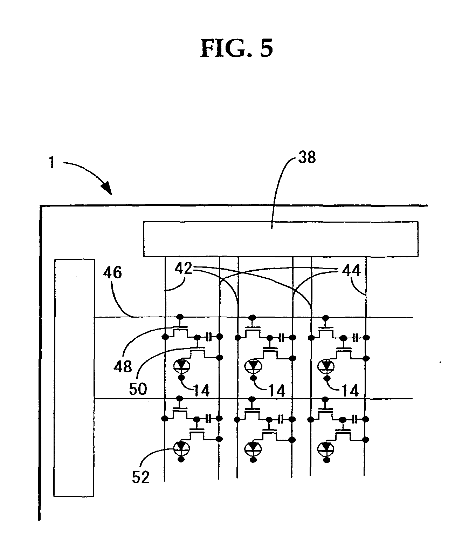

In the aforementioned active matrix panel, as shown in FIG.5, plural scanning lines 46 are arranged in parallel to each other, intersecting in substantially right angles with plural data lines 42 and current supply lines 44, which are parallel to each other, to form grids, and a switching TFT 48 and driver TFT 50 are connected to each grid to form a circuit. If a current is applied from a driver circuit 38, the switching TFT 48 and driver TFT 50 can be driven for each grid. In each grid, the organic thin film elements 24, 26, and 28 for blue light emission, green light emission and red light emission function as a pixel. In this active matrix panel, if a current is applied from the drive circuit 38 to one of the scanning lines 46 arranged in the horizontal direction, and the data line 42 arranged in the vertical direction, the switching TFT 48 situated at the intersection is driven, the driver TFT 50 is driven as a result, and an organic EL element 52 at this position emits light by a current from the current supply line 44. By controlling the light emission of this pixel unit, a full color picture can easily be formed.

The organic EL display of the present invention may be conveniently used in fields of various kinds such as computers, vehicle-mounted display devices, field-ready display devices, home apparatuses, industrial apparatuses, household electric appliances, traffic display devices, clock display devices, calendar display units, luminescent screens and audio equipment.

Hereinafter, specific examples of the present invention will be described, but it should be understood that the present invention is not limited to these examples.

### (Example 1)

### - Synthesis of 1,3,6,8-tetrakis(N-(3-methylphenyl)-N-phenylamino)pyrene -

1,3,6,8-tetrabromopyrene was synthesized by the reaction of pyrene and bromine based on the method stated in *Annalen der Chemie,* Vol. 531, p.81. 1,3,6,8-tetrabromopyrene was then diarylaminated. The diarylanimation was performed according to a general method of triarylamine synthesis from an aryl halide, which is stated in Tetrahedron Letters, Vol.39, p. 2367 (1998). Specifically, 4 equivalents of 3-methyldiphenylamine, 4 equivalents of sodium-t-butoxide, 0.1 % equivalents of palladium acetate and 0.4% equivalents of tri(t-butyl)phosphine were reacted with 1,3,6,8-tetrabromopyrene using o-xylene as a solvent at 130°C for 3 hours. After the reaction was complete, the product was cooled, the solution used for the reaction was washed several times with water, and the o-xylene was distilled off. The remaining oily matter was washed with methanol, and a crude reaction product was obtained by recrystallizing the oily matter with THF-methanol. By purifying the crude reaction product by vacuum sublimation, 1,3,6,8-tetrakis(N-(3-methylphenyl)-N-phenylamino)pyrene was obtained.
1,3,6,8-tetrakis(N-(3-methylphenyl)-N-phenylamino)pyrene is a compound where, in the formula (1), R¹ to R⁴ are the groups represented by the following formula:

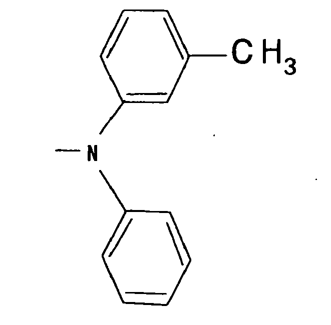

The result of the mass spectrum analysis, elemental analysis and IR analysis of the thus synthesized 1,3,6,8-tetrakis (N-(3-methylphenyl)-N-phenylamino)pyrene, are shown below.

### <Mass spectrum>

Using the formula C₆₈H₅₄N₄ and atomic weights of 12 for carbon, 1 for hydrogen, and 14 for nitrogen, the calculated molecular weight was 926.

Molecular weight peaks in the mass spectrum were at 926 and 927.

### <Elemental analysis>

| Using the formula C₆₈H₅₄N₄; | | | |
|---|---|---|---|
| Calculated value: | C: 88.09%, | H: 5.87%, | N: 6.04% |
| Experimental value: | C: 88.40%, | H: 5.94%, | N: 6.12% |

### <IR analysis>

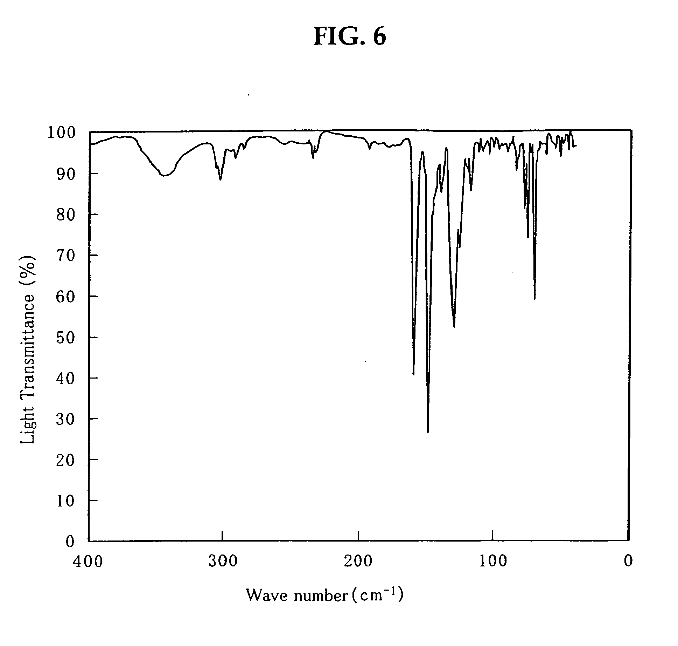

The IR spectrum of the synthesized 1,3,6,8-tetrakis(N-(3-methylphenyl)-N-phenylamino)pyrene taken by the KBr pellet method is shown in FIG. 6.

### (Example 2)

### - Synthesis of 1,3,6,8-tetrakis[N-(1-naphthyl)-N-phenylamino]pyrene-

1,3,6,8-tetrakis(N-(1-naphthyl)-N-phenylamino)pyrene was synthesized as in Example 1, except that 3-methyldiphenylamine was replaced by (1-naphthyl)-N-phenylamine. 1,3,6,8-tetrakis[N-(1-naphthyl)-N-phenylamino]pyrene is a compound, where, in the formula (1), R¹ to R⁴ are the groups represented by the following formula:

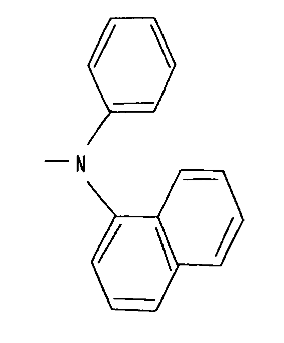

The mass spectrum analysis, the elemental analysis and the IR analysis of the thus synthesized 1,3,6,8-tetrakis[N-(1-naphthyl)-N-phenylamino]pyrene, are shown below.

### <Mass spectrum>

Using the formula C₈₀H₅₄N₄ and atomic weights of 12 for carbon, 1 for hydrogen, and 14 for nitrogen, the calculated molecular weight was 1070.

Molecular weight peaks in the mass spectrum were at 1070 and 1071.

### <Elemental analysis>

| Using the formula C₈₀H₅₄N₄; | | | |
|---|---|---|---|
| Calculated value | C:89.69%, | H:5.08%, | N:5.23% |
| Experimental value | C:90.09%, | H:5.28%, | N:5.07% |

### <IR analysis>

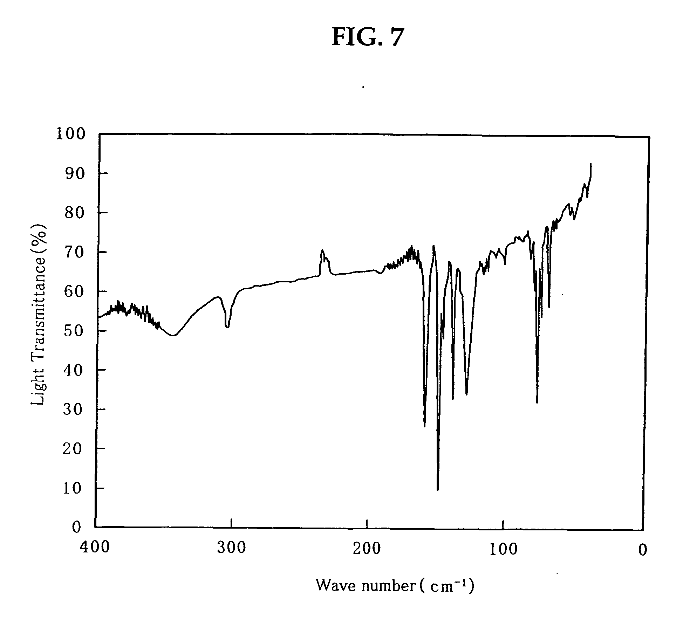

The IR spectrum of the synthesized 1,3,6,8-tetrakis[N-(1-naphthyl)-N-phenylamino]pyrene by the KBr pellet method is shown in FIG. 7.

### (Example 3)

### - Synthesis of 1,3,6,8-tetrakis [4,4'-bis(α,α-dimethylbenzyl)diphenylamino]pyrene -

1,3,6,8-tetrakis[4,4'-bis(α,α-dimethylbenzyl)diphenylamino]pyrene was synthesized as in Example 1, except that
3-methyldiphenylamine was replaced by 4,4'-bis(α,α-dimethylbenzyl)diphenylamine.
1,3,6,8-tetrakis[4,4'-bis (α,α-dimethylbenzyl)diphenylamino]pyrene is a compound where, in the formula (1), R¹ to R⁴ are the groups represented by the following formula:

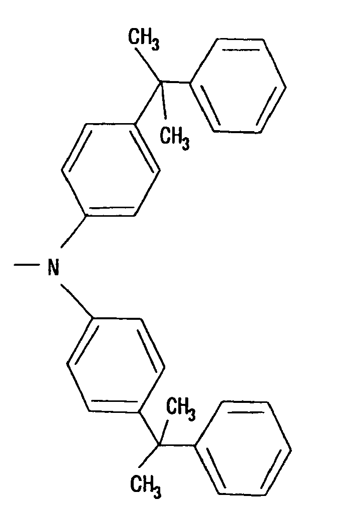

### (Example 4)

### - Manufacture of Organic EL Element -

A laminated type organic EL element using the 1,3,6,8-tetrakis[N-(3-methylphenyl)-N-phenylamino]pyrene synthesized in Example 1 as the luminescent material of the light-emitting layer, was manufactured as follows. A glass substrate on which an ITO electrode was formed as a positive electrode, was subjected to ultrasonic cleaning in water, acetone and isopropyl alcohol, and then was given UV ozonization. Thereafter, N,N'-dinaphthyl-N,N'-diphenyl-[1,1'-biphenyl]-4,4'-diamine (NPD) was coated to a thickness of 50nm as a positive hole transport layer on the ITO electrode using a vacuum vapor deposition device (degree of vacuum = 1x10⁻⁶Torr (1.3x10⁻⁴Pa), temperature of the substrate = room temperature). Next, a light-emitting layer of 1,3,6,8-tetrakis(N-(3-methylphenyl)-N-phenylamino)pyrene was coated by deposition on the positive hole transport layer of N,N'-dinaphthyl-N,N'-diphenyl-[1,1'-biphenyl]-4,4'-diamine (NPD) to a thickness of 20nm, and
2,9-dimethyl-4,7-diphenyl-1,10-phenanthroline (BCP) was coated by deposition as a first electron transport layer on the light-emitting layer to a thickness of 10nm. Thereafter, aluminium quinoline complex (Alq) was coated by deposition as a second electron transport layer on the first electron transport layer to a thickness of 20nm, and Al-Li alloy (content of Li = 0.5 % by mass) was provided by vapor-deposition as a negative electrode on the second electron transport layer of aluminium quinoline complex (Alq) to a thickness of 50nm. In this way, the organic EL element was manufactured.

When a voltage was applied to the ITO electrode (positive electrode) and Al-Li alloy (negative electrode) in the thus manufactured organic EL element, in this organic EL element, a green light emission was observed at a voltage of 5V or higher, and a high color purity green light emission with a emission luminance of 1,920 cd/m² was observed at an applied voltage of 10V.

### (Example 5)

### - Manufacture of Organic EL Element -

An organic EL element was manufactured as in Example 4, except that the light-emitting layer was formed by simultaneous vapor deposition of
1,3,6,8-tetrakis(N-(3-methylphenyl)-N-phenylamino)pyrene and N,N'-dinaphthyl-N,N'-diphenyl-[1,1'-biphenyl]-4,4'-diamine (NPD), so that there were 99 molecules of NPD (99 moles) to each molecule (one mole) of
1,3,6,8-tetrakis(N-(3-methylphenyl)-N-phenylamino)pyrene.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 2,850cd/m² was observed at an applied voltage of 10V.

### (Example 6)

### - Manufacture of Organic EL element -

An organic EL element was manufactured as in Example 5, except that
N,N'-dinaphthyl-N,N'-diphenyl-[1,1'-biphenyl]-4,4'-diamine (NPD) was replaced by 4,4'-bis (9-carbazolyl)-biphenyl (CBP) as a material for the light-emitting layer.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 2,600 cd/m² was observed at an applied voltage of 10V.

### (Example 7)

### - Manufacture of Organic EL element -

An organic EL element was manufactured as in Example 4, except that
1,3,6,8-tetrakis(N-(3-methylphenyl)-N-phenylamino)pyrene synthesized in Example 1 was replaced by 1,3,6,8-tetrakis [N-(1-naphthyl)-N-phenylamino] pyrene synthesized in Example 2.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 2,010cd/m² was observed at an applied voltage of 10V.

### (Example 8)

### - Manufacture of Organic EL element -

An organic EL element was manufactured as in Example 5, except that 1,3,6,8-tetrakis
[N-(3-methylphenyl)-N-phenylamino]pyrene synthesized in Example 1, was replaced by
1,3,6,8-tetrakis[N-(1-naphthyl)-N-phenylamino]pyrene synthesized in Example 2.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 2,900cd/m² was observed at an applied voltage of 10V.

### (Example 9)

### - Manufacture of Organic EL element -

An organic EL element was manufactured as in Example 7, except that a positive hole transport and light-emitting layer (thickness: 50nm) was formed using the
1,3,6,8-tetrakis(N-(1-naphthyl)-N-phenylamino)pyrene instead of forming the positive hole transport layer and the light-emitting layer and that the thickness of the first and second electron transport layers were increased by 10 nm each to 20 nm and 30 nm, respectively.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 1,800cd/m² was observed at an applied voltage of 10V.

### (Example 10)

### - Manufacture of Organic EL element -

An organic EL element was manufactured as in Example 5, except that
1,3,6,8-tetrakis(N-(3-methylphenyl)-N-phenylamino)pyrene synthesized in Example 1 was replaced by
1,3,6,8-tetrakis[4,4'-bis(α,α-dimethyl benzyl)diphenylamino]pyrene synthesized in Example 3.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 3,000cd/m² was observed at an applied voltage of 10V.

### (Example 11)

### - Manufacture of Organic EL element -

An organic EL element was manufactured as in Example 5, except that
N,N'-dinaphthyl-N,N'-diphenyl-[1,1'-biphenyl]-4,4'-diamine (NPD) was replaced by aluminium quinoline complex (Alq) as the material of the light-emitting layer, and the first electron transport layer of BCP was omitted.

When a voltage was applied to the ITO electrode (positive electrode) and Al-Li alloy (negative electrode) in the manufactured organic EL element, in this organic EL element, a green light emission was observed at a voltage of 5V or higher, and a high color purity green light emission with a emission luminance of 2,620cd/m² was observed at an applied voltage of 10V.

The present invention resolves the problems in the related art, and provides the 1,3,6,8-tetrasubstituted pyrene compound mentioned in claim 1 as a material of a green light emission in an organic EL element, an organic EL element having excellent light-emitting efficiency, emission luminance and color purity of green light, and a high performance organic EL display using the organic EL element.

## Claims

1. An organic EL element comprising:
an organic thin film layer between a positive electrode and a negative electrode, the organic thin film layer containing a 1,3,6,8-tetrasubstituted pyrene compound represented by the following formula (1) as a luminescent material: 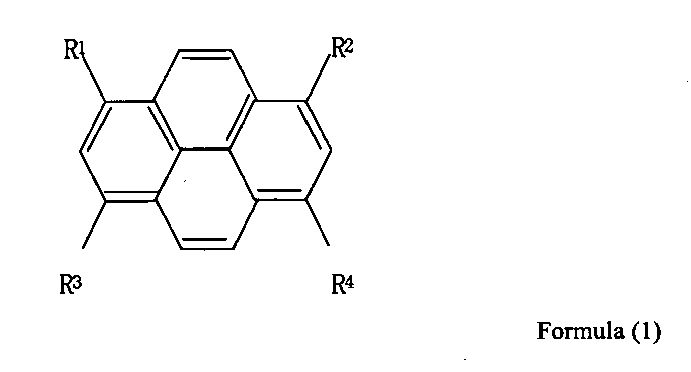 wherein R¹ to R⁴ may be the same or different, and represent a group represented by the following formula (2): 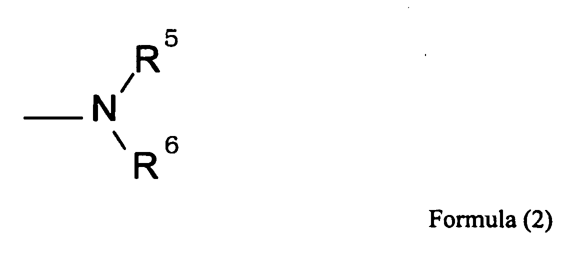 wherein R⁵ and R⁶ may be the same or different, and represent an aryl group wherein R⁵ and R⁶ do not form a ring by bonding.

2. An organic EL element according to Claim 1, wherein R¹ to R⁴ are groups represented by the following formula (3), and the 1,3,6,8-tetrasubstituted pyrene compound is 1,3,6,8-tetrakis (N,N'-diphenylamino) pyrene: 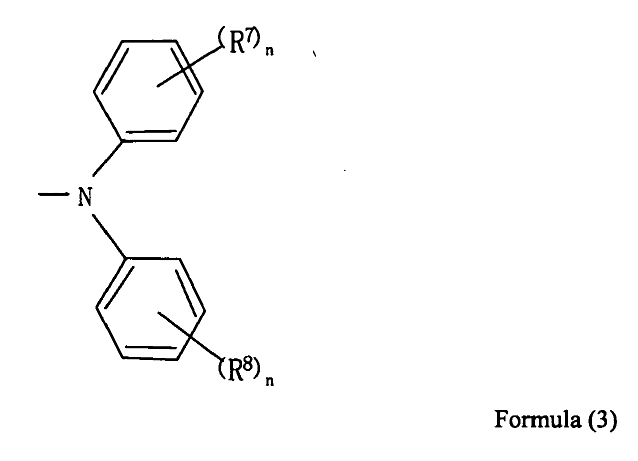 wherein R⁷ and R⁸ may be the same or different, and each represent at least one hydrogen atom, at least one alkyl group, or at least one aryl group; and the letter "n" represents an integer of 1 or more.

3. An organic EL element according to Claim 1, wherein R¹ to R⁴ are groups represented by the following formula (4), and the 1,3,6,8-tetrasubstituted pyrene compound is 1,3,6,8-tetrakis [N-(1-naphthyl)-N-phenyl amino]pyrene: 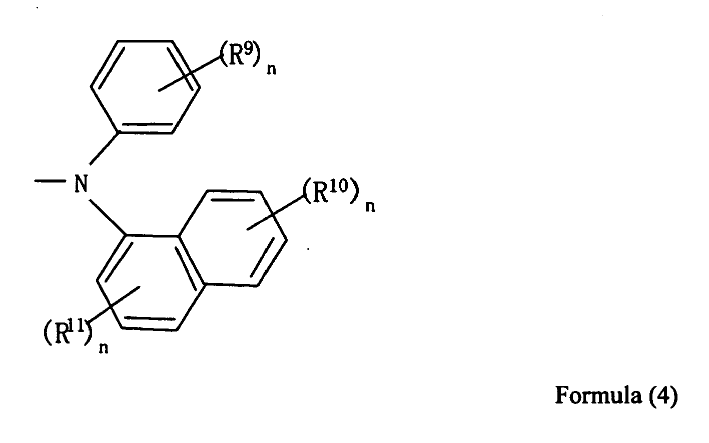 wherein R⁹, R¹⁰ and R¹¹ may be the same or different, and each represent at least one hydrogen atom, at least one alkyl group, or at least one aryl group; and the letter "n" represents an integer of 1 or more..

4. An organic EL element according to Claim 1, wherein R¹ to R⁴ are groups represented by the following formula (5), and the 1,3,6,8-tetrasubstituted pyrene compound is 1,3,6,8-tetrakis [4,4'-bis(α,α-dimethylbenzyl)diphenylamino]pyrene: 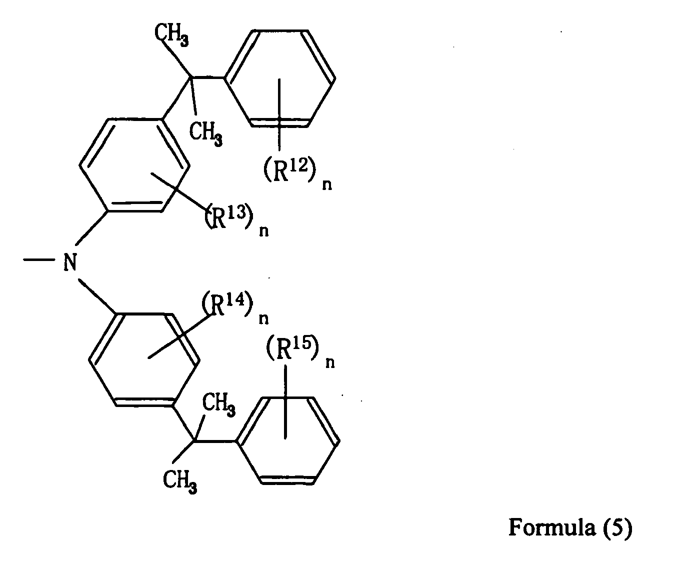 wherein R¹², R¹³, R¹⁴ and R¹⁵ may be the same or different, and each represent at least one hydrogen atom, at least one alkyl group, or at least one aryl group; and the letter "n" represents an integer of 1 or more.

5. An organic EL element according to any one of Claims 1 to 4, wherein the organic thin film layer comprises a light-emitting and electron transport layer containing the 1,3,6,8-tetrasubstituted pyrene compound as a luminescent material.

6. An organic EL element according to any one of Claims 1 to 4, wherein the organic thin film layer comprises a light-emitting layer between a positive hole transport layer and an electron transport layer, the light-emitting layer containing the 1,3,6,8-tetrasubstituted pyrene compound as a luminescent material.

7. An organic EL element according to Claim 6, wherein the light-emitting layer consists of the 1,3,6,8-tetrasubstituted pyrene compound represented by the formula (1).

8. An organic EL element according to any one of Claims 5 to 7, wherein the light-emitting layer comprises an aromatic amine derivative represented by the following formula (6): 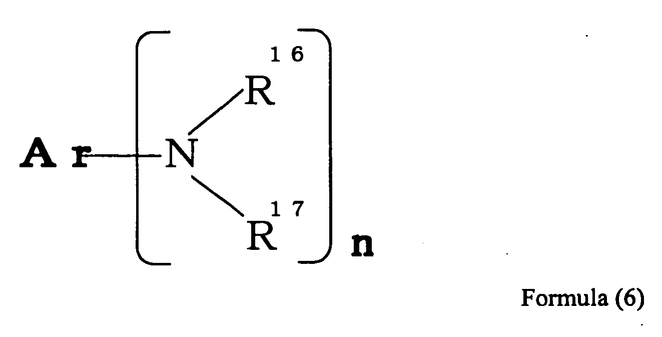 wherein "n" is 2 or 3, "Ar" represents a group selected from the group consisting of divalent aromatic group, trivalent aromatic group, divalent heterocylic aromatic group, and trivalent heterocyclic aromatic group, and R¹⁶ and R¹⁷ may be the same or different representing one of a monovalent aromatic group and a heterocyclic aromatic group.

9. An organic EL element according to Claim 8, wherein the aromatic amine derivative is selected from N,N'-dinaphthyl-N,N'-diphenyl-[1,1'-biphenyl]-4,4'-diamine (NPD) represented by the following formula (7) and a derivative thereof:

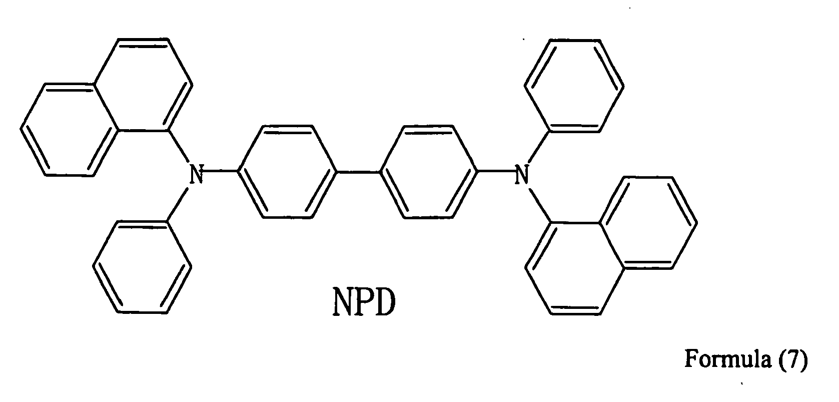

10. An organic EL element according to any one of Claims 5 to 9, wherein the light-emitting layer comprises a carbazole derivative represented by the following formula (8): 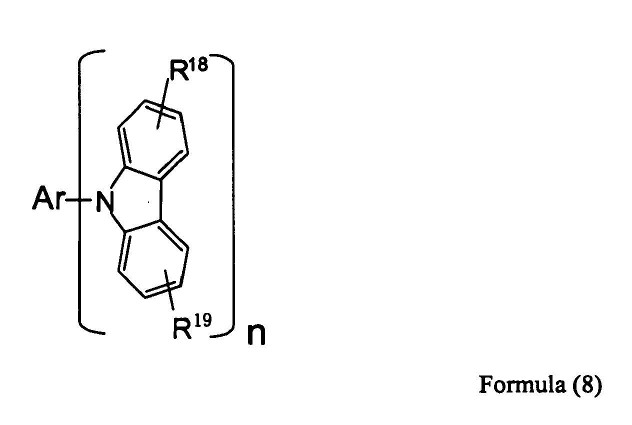 wherein "Ar" represents a group selected from the group of a divalent group having an aromatic ring, a trivalent group having an aromatic group, a divalent group having a heterocyclic aromatic ring, and a trivalent group having a heterocyclic ring; R¹⁸ and R¹⁹ each indepently represent a hydrogen atom, a halogen atom, a substituted or unsubstituted alkyl group, a substituted or unsubstituted aralkyl group, a substituted or unsubstituted alkenyl group, a substituted or unsubstituted aryl group, a substituted or unsubstituted cyano group, a substituted or unsubstituted amino group, a substituted or unsubstituted acyl group, a substituted or unsubstituted alkoxy carbonyl group, a substituted or unsubstituted carboxyl group, a substituted or unsubstituted alkoxy group, a substituted or unsubstituted alkyl sulfonyl group, a substituted or unsubstituted hydroxyl group, a substituted or unsubstituted amide group, a substituted or unsubstituted aryloxy group, a substituted or unsubstituted aromatic hydrocarbon ring, or a substituted or unsubstituted aromatic heterocyclic group; and "n" is an integer of 2 or 3.

11. An organic EL element according to Claim 10, wherein the carbazole derivative is selected from 4,4'-bis(9-carbazolyl)-biphenyl (CBP) represented by the following formula (9) and a derivative thereof:

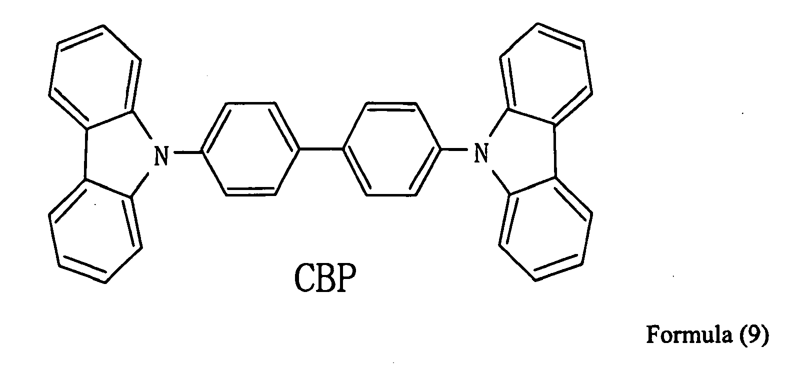

12. An organic EL element according to any one of Claims 5 to 11, wherein the light-emitting layer comprises an oxine complex represented by the following formula (10): 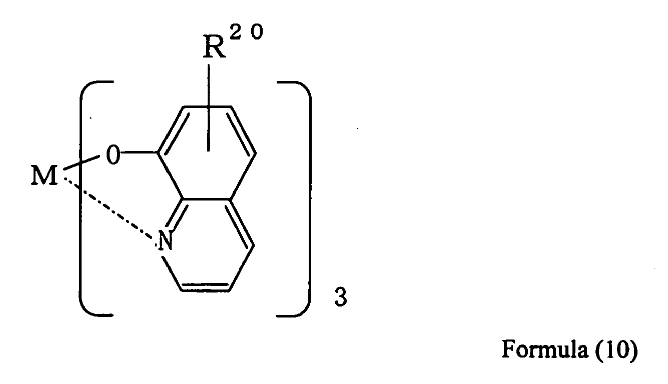 wherein "M" represents a metal atom, and R²⁰ represents a hydrogen atom, halogen atom, alkyl group, aralkyl group, alkenyl group, aryl group, cyano group, amino group, acyl group, alkoxycarbonyl group, carboxyl group, alkoxy group, alkylsulfonyl group, hydroxyl group, amide group, aryloxy group, aromatic hydrocarbon ring or aromatic heterocyclic group. These may be further substituted by substituents.

13. An organic EL element according to Claim 12, wherein the oxine complex is aluminium quinoline complex (Alq) represented by the following formula (11):

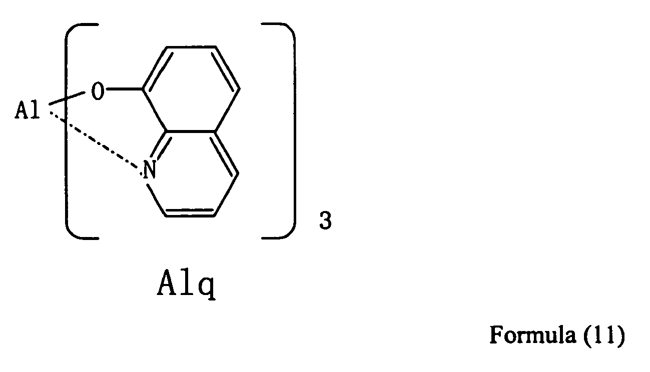

14. An organic EL element according any one of Claims 5 to 13, wherein the electron transport layer comprises 2,9-dimethyl-4,7-diphenyl-1,10-phenanthroline (BCP) represented by the following formula (12) as an electron transport material:

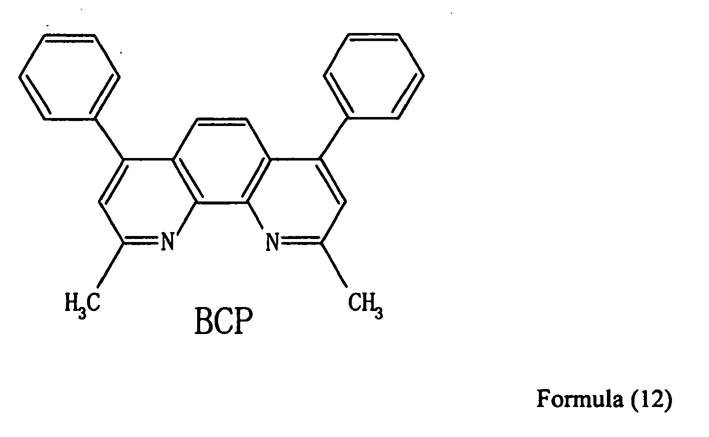

15. An organic EL element according to any one of Claims 1 to 14, wherein the organic EL element emits green light.

16. An 1,3,6,8-tetrasubstituted pyrene compound represented by the following formula (1) 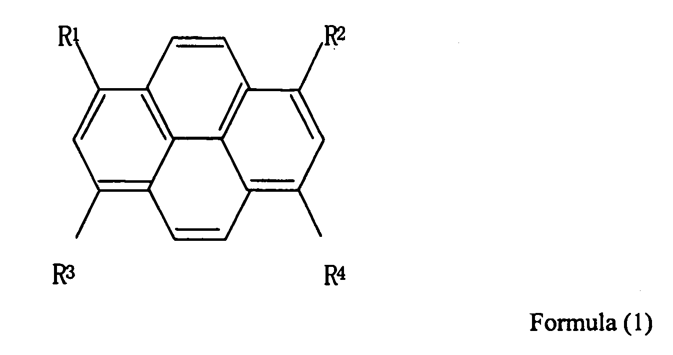 wherein R¹ to R⁴ are groups represented by the following formula (4), and the 1,3,6,8-tetrasubstituted pyrene compound is 1,3,6,8-tetrakis [N-(1-naphthyl)-N-phenylamino]pyrene: 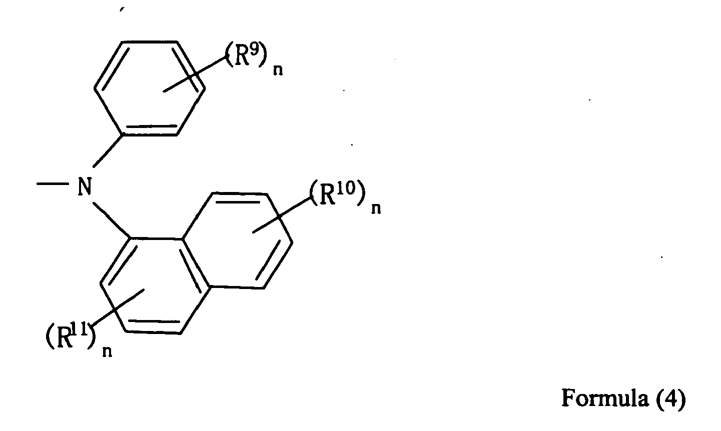 wherein R⁹, R¹⁰ and R¹¹ may be the same or different, and each represent at least one hydrogen atom, at least one alkyl group, or at least one aryl group; and the letter "n" represents an integer of 1 or more.

17. An 1,3,6,8-tetrasubstituted pyrene compound represented by the following formula (1) 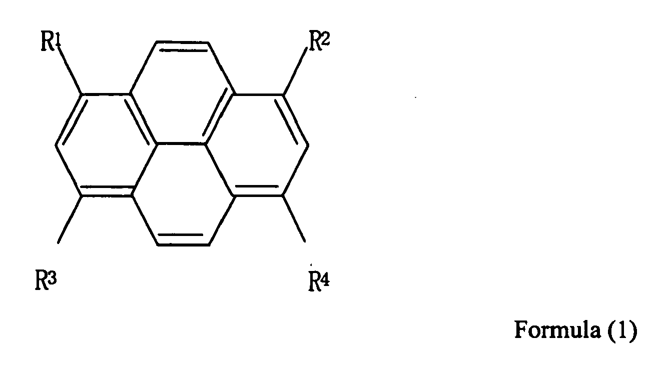 wherein R¹ to R⁴ are groups represented by the following formula (5), and the 1,3,6,8-tetrasubstituted pyrene compound is 1,3,6,8-tetrakis [4,4'-bis (α,α-dimethylbenzyl)diphenylamino]pyrene: 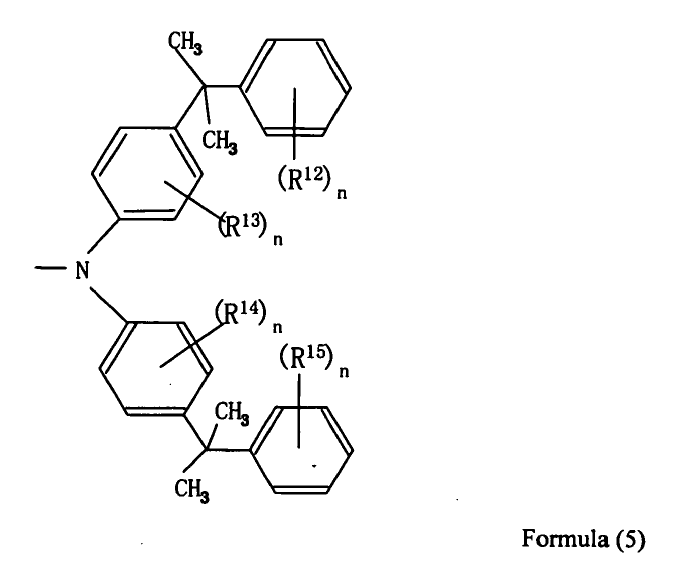 wherein R¹², R¹³, R¹⁴ and R¹⁵ may be the same or different, and each represent at least one hydrogen atom, at least one alkyl group, or at least one aryl group; and the letter "n" represents an integer of 1 or more.

18. An 1,3,6,8-tetrasubstituted pyrene compound according to any one of Claims 16 to 17, used as a luminescent material in an organic EL element.

19. An organic EL display, comprising an organic EL element according to any one of Claims 1 to 15.

20. An organic EL display according to Claim 19, wherein the organic display is one of a passive matrix panel and an active matrix panel.

## Patentansprüche

1. Organisches Elektrolumineszenz (EL) Element mit:
einer organischen Dünnfilmschicht zwischen einer positiven Elektrode und einer negativen Elektrode, wobei die organische Dünnfilmschicht als Leuchtstoff eine 1,3,6,8-tetrasubstituierte Pyrenverbindung enthält, welche durch die folgende Formel (1) dargestellt ist: 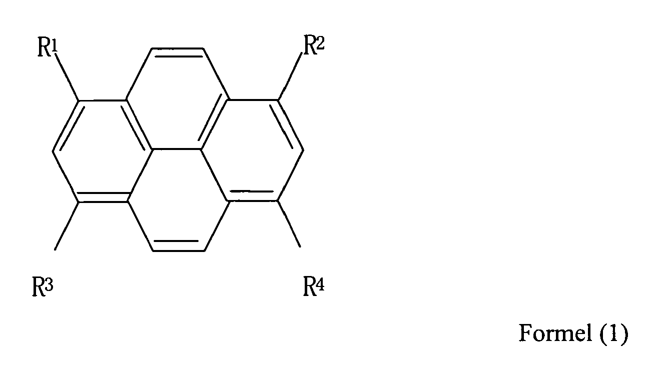 wobei R¹ bis R⁴ gleich oder unterschiedlich sein können und eine durch die folgende Formel (2) dargestellte Gruppe darstellen: 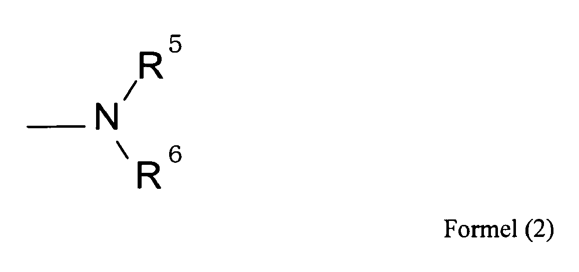 wobei R⁵ und R⁶ gleich oder unterschiedlich sein können und eine Arylgruppe darstellen, wobei R⁵ and R⁶ keinen Ring durch Bindung ausbilden.

2. Organisches EL Element gemäß Anspruch 1, wobei R¹ bis R⁴ aus durch die folgende Formel (3) dargestellten Gruppen bestehen, und die 1,3,6,8-tetrasubstituierte Pyrenverbindung aus 1,3,6,8-Tetrakis (N,N'-Diphenylamino) Pyren besteht: 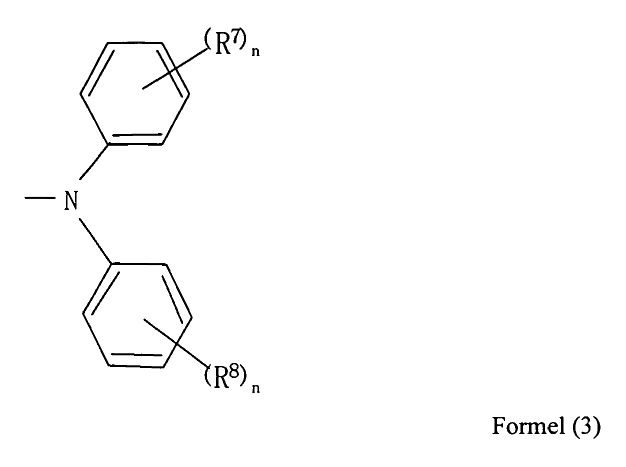 wobei R⁷ und R⁸ gleich oder unterschiedlich sein können und jeweils mindestens ein Wasserstoffatom, mindestens eine Alkylgruppe oder mindestens eine Arylgruppe darstellen; und der Buchstabe "n" eine ganze Zahl von 1 oder mehr darstellt.

3. Organisches EL Element gemäß Anspruch 1, wobei R¹ bis R⁴ aus durch die folgende Formel (4) dargestellten Gruppen bestehen und die 1,3,6,8-tetrasubstituierte Pyrenverbindung aus 1,3,6,8-Tetrakis [N-(1-Naphthyl)-N-Phenylamino]Pyren besteht: 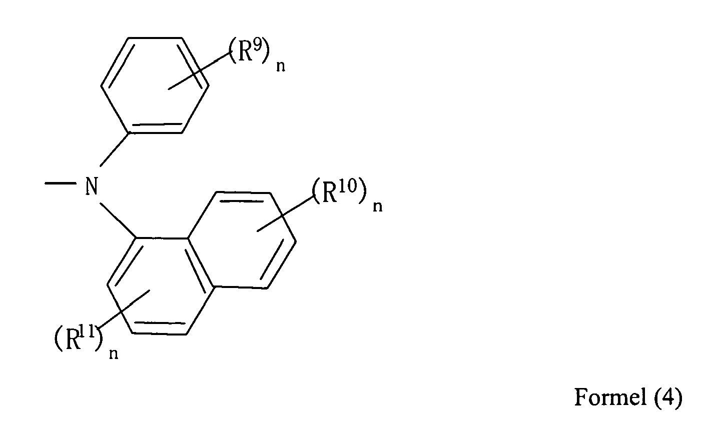 wobei R⁹, R¹⁰ und R¹¹ gleich oder unterschiedlich sein können und jeweils mindestens ein Wasserstoffatom, mindestens eine Alkylgruppe oder mindestens eine Arylgruppe darstellen; und der Buchstabe "n" eine ganze Zahl von ein 1 oder mehr darstellt.

4. Organisches EL Element gemäß Anspruch 1, wobei R¹ bis R⁴ aus durch die folgende Formel (5) darstellten Gruppen bestehen und die 1,3,6,8-tetrasubstituierte Pyrenverbindung aus 1,3,6,8-Tetrakis [4,4'-bis(α,α-Dimethylbenzyl)Diphenylamino]Pyren besteht: 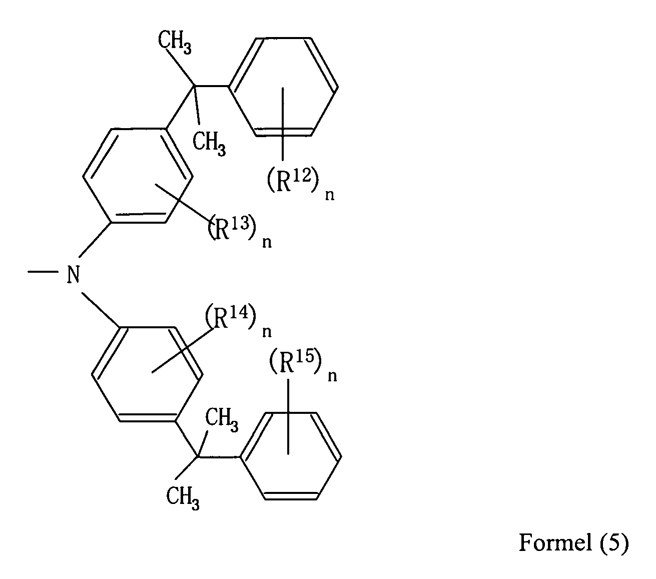 wobei R¹², R¹³, R¹⁴ und R¹⁵ gleich oder unterschiedlich sein können und jeweils mindestens ein Wasserstoffatom, mindestens eine Alkylgruppe oder mindestens eine Arylgruppe darstellen; und der Buchstabe "n" eine ganze Zahl von 1 oder mehr darstellt.

5. Organisches EL Element gemäß einem der Ansprüche 1 bis 4, wobei die organische Dünnfilmschicht eine lichtemittierende und Elektronentransportschicht umfasst, die die 1,3,6,8-tetrasubstituierte Pyrenverbindung als Leuchtstoff enthält.

6. Organisches EL Element gemäß einem der Ansprüche 1 bis 4, wobei die organische Dünnfilmschicht eine lichtemittierende Schicht zwischen einer positiven Lochtransportschicht und einer Elektronentransportschicht umfasst, wobei die lichtemittierende Schicht die 1,3,6,8-tetrasubstituierte Pyrenverbindung als Leuchtstoff enthält.

7. Organisches EL Element gemäß Anspruch 6, wobei die lichtemittierende Schicht aus der 1,3,6,8-tetrasubstituierten Pyrenverbindung besteht, die durch die Formel (1) dargestellt wird.

8. Organisches EL Element gemäß einem der Ansprüche 5 bis 7, wobei die lichtemittierende Schicht ein aromatisches Aminderivat umfasst, das durch die folgende Formel (6) dargestellt wird: 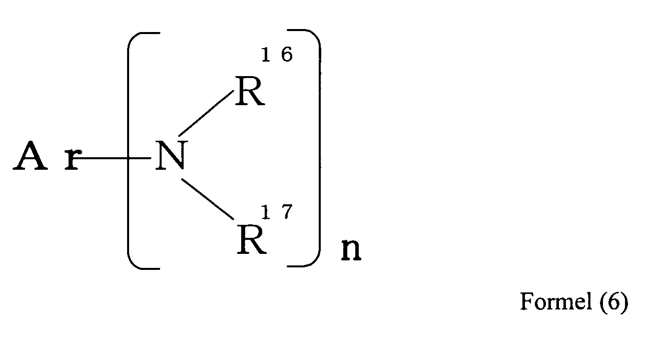 wobei "n" 2 oder 3 ist, "Ar" eine aus der Gruppe bestehend aus einer zweiwertigen aromatischen Gruppe, einer dreiwertigen aromatischen Gruppe, einer zweiwertigen heterozyklischen aromatischen Gruppe und einer dreiwertigen heterozyklischen Gruppe ausgewählte Gruppe darstellt und R¹⁶ und R¹⁷ gleich oder unterschiedlich sein können und entweder eine einwertige aromatische Gruppe oder eine heterozyklische aromatische Gruppe darstellen.

9. Organisches EL Element gemäß Anspruch 8, wobei das aromatische Aminderivat aus dem durch die folgende Formel (7) dargestellten N,N'-Dinaphthyl-N,N'-Diphenyl-[1,1'-Biphenyl]-4,4'-Diamin (NPD) sowie einem Derivat von diesem ausgewählt ist:

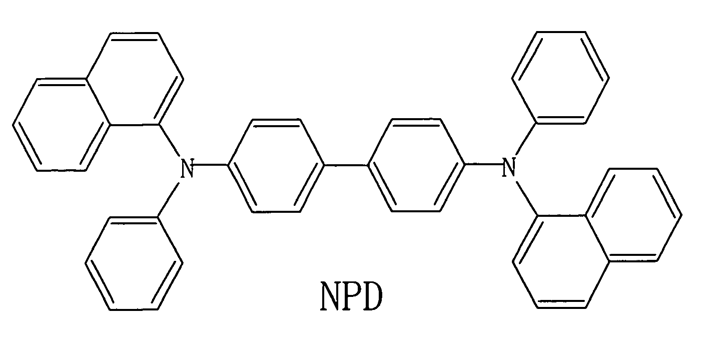

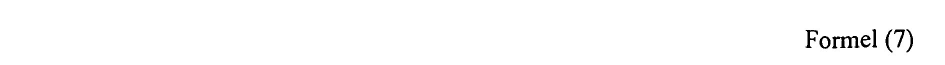

10. Organisches EL Element gemäß einem der Ansprüche 5 bis 9, wobei die lichtemittierende Schicht ein durch die folgende Formel (8) dargestelltes Karbazolderivat umfasst: 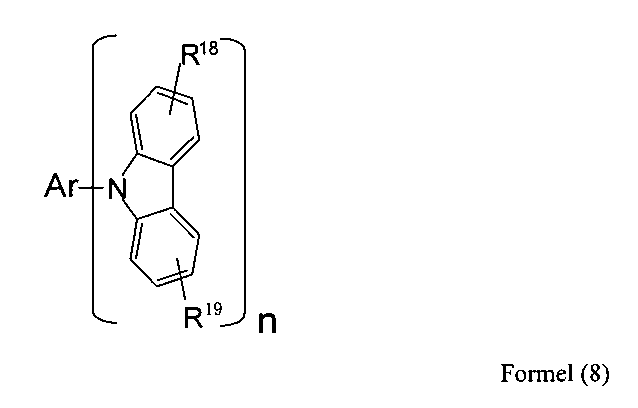 wobei "Ar" eine Gruppe ausgewählt aus der Gruppe einer zweiwertigen Gruppe mit einem aromatischen Ring, einer dreiwertigen Gruppe mit einer aromatischen Gruppe, einer zweiwertigen Gruppe mit einem heterozyklischen aromatischen Ring sowie einer dreiwertigen Gruppe mit einem heterozyklischen Ring darstellt; R¹⁸ und R¹⁹ stellen jeweils unabhängig voneinander ein Wasserstoffatom, ein Halogenatom, eine substituierte oder unsubstituierte Alkylgruppe, eine substituierte oder unsubstituierte Aralkylgruppe, eine substituierte oder unsubstituierte Alkenylgruppe, eine substituierte oder unsubstituierte Arylgruppe, eine substituierte oder unsubstituierte Zyangruppe, eine substituierte oder unsubstituierte Aminogruppe, eine substituierte oder unsubstituierte Azylgruppe, eine substituierte oder unsubstituierte Alkoxylkarbonylgruppe, eine substituierte oder unsubstituierte Karboxylgruppe, eine substituierte oder unsubstituierte Alkoxylgruppe, eine substituierte oder unsubstituierte Alkylsulfonylgruppe, eine substituierte oder unsubstituierte Hydroxygruppe, eine substituierte oder unsubstituierte Amidgruppe, eine substituierte oder unsubstituierte Aryloxygruppe, einen substituierten oder unsubstituierten aromatischen Kohlenwasserstoffring oder eine substituierte oder unsubstituierte aromatische heterozyklische Gruppe darstellt; und "n" eine ganze Zahl von 2 oder 3 ist.

11. Organisches EL Element gemäß Anspruch 10, wobei das Karbazolderivat aus durch die Formel (9) dargestelltem 4,4'-bis(9-Karbazolyl)-Biphenyl (CBP) sowie einem Derivat von diesem ausgewählt ist:

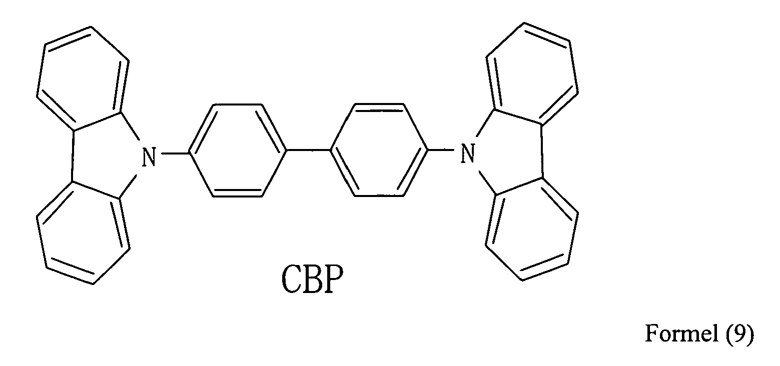

12. Organisches EL Element gemäß einem der Ansprüche 5 bis 11, wobei die lichtemittierende Schicht einen Oxinkomplex umfasst, der durch die folgende Formel (10) dargestellt ist: 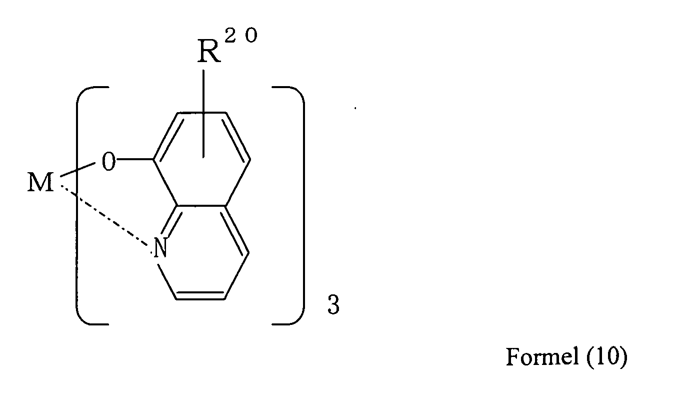 wobei "M" ein Metallatom darstellt und R²⁰ ein Wasserstoffatom, Halogenatom, eine Alkylgruppe, Aralkylgruppe, Alkenylgruppe, Arylgruppe, Zyangruppe, Aminogruppe, Azylgruppe, Alkoxylkarbonylgruppe, Karboxylgruppe, Alkoxylgruppe, Alkylsulfonylgruppe, Hydroxygruppe, Amidgruppe, Aryloxylgruppe, einen aromatischen Kohlenwasserstoffring oder eine aromatische heterozyklische Gruppe darstellt, welche weiterhin durch Substituenten substituiert werden können.

13. Organisches EL Element gemäß Anspruch 12, wobei der Oxinkomplex aus einem durch die Formel (11) dargestellten Aluminiumchinolinkomplex (Alq) besteht:

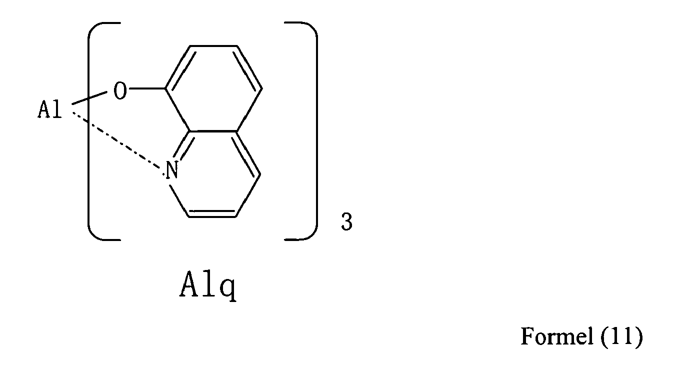

14. Organisches EL Element gemäß einem der Ansprüche 5 bis 13, wobei die Elektronentransportschicht durch die folgende Formel (12) dargestelltes 2,9-Dimethyl-4,7-Diphenyl-1,10-Phenanthrolin (BCP) als ein Elektronentransportmaterial umfasst:

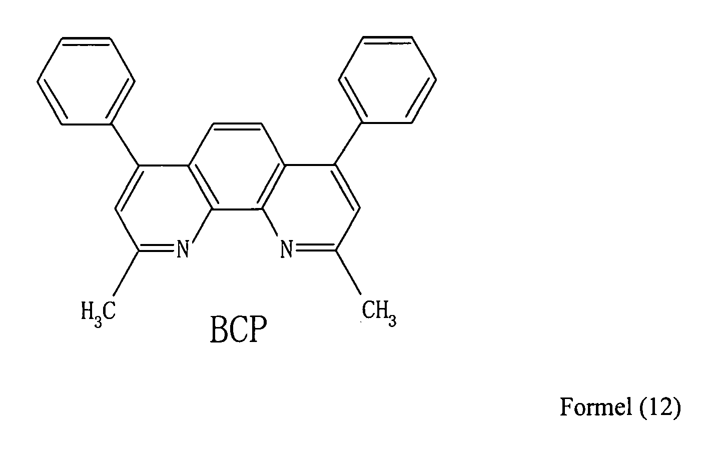

15. Organisches EL Element gemäß einem der Ansprüche 1 bis 14, wobei das organische EL Element grünes Licht emittiert.

16. Eine durch die folgende Formel (1) dargestellte 1,3,6,8-tetrasubstituierte Pyrenverbindung 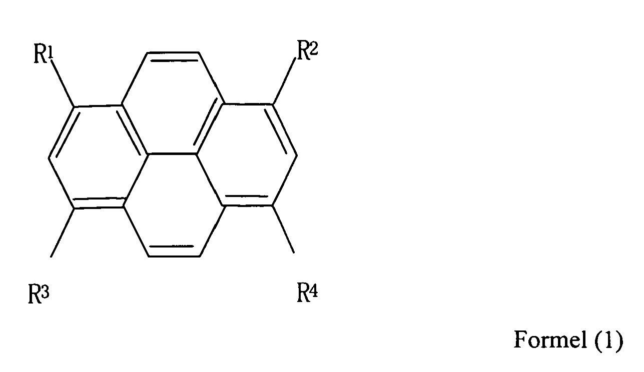 wobei R¹ bis R⁴ aus durch die folgende Formel (4) dargestellten Gruppen bestehen und die 1,3,6,8-tetrasubstituierte Pyrenverbindung aus 1,3,6,8-Tetrakis [N-(1-Naphthyl)-N-Phenylamino]Pyren besteht: 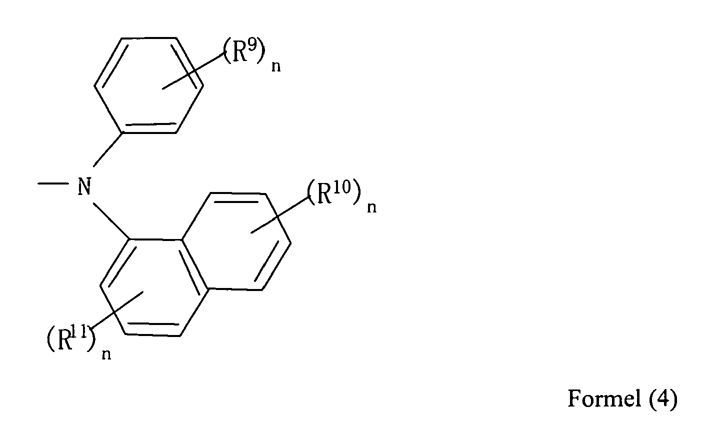 wobei R⁹, R¹⁰ und R¹¹ gleich oder unterschiedlich sein können und jeweils mindestens ein Wasserstoffatom, mindestens eine Alkylgruppe oder mindestens eine Arylgruppe darstellen; und der Buchstabe "n" eine ganze Zahl von 1 oder mehr darstellt.

17. Eine durch die folgende Formel (1) dargestellte 1,3,6,8-tetrasubstituierte Pyrenverbindung 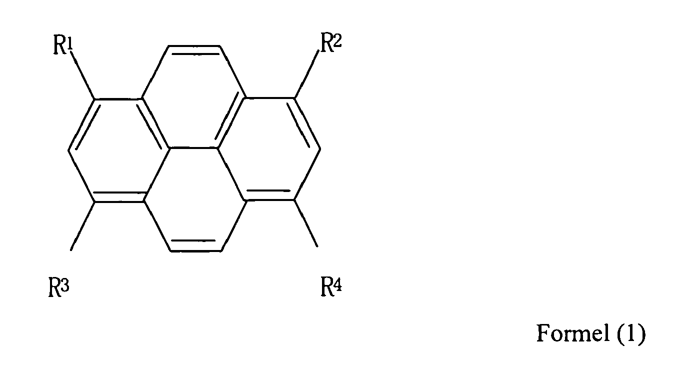 wobei R¹ bis R⁴ aus durch die folgende Formel (5) dargestellten Gruppen bestehen und die 1,3,6,8-tetrasubstituierte Pyrenverbindung aus 1,3,6,8-Tetrakis [4,4'-bis (α,α-Dimethylbenzyl)Diphenylamino]Pyren besteht: 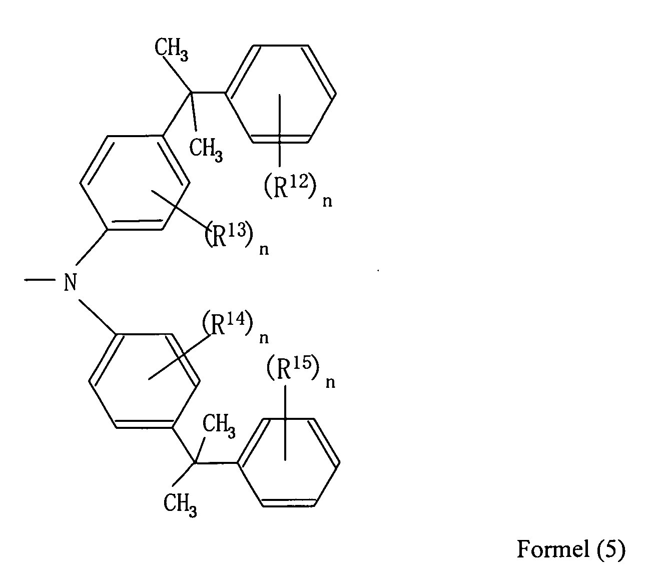 wobei R¹², R¹³, R¹⁴ und R¹⁵ gleich oder unterschiedlich sein können und jeweils mindestens ein Wasserstoffatom, mindestens eine Alkylgruppe oder mindestens eine Arylgruppe darstellen; und der Buchstabe "n" eine ganze Zahl von 1 oder mehr darstellt.

18. Eine 1,3,6,8-tetrasubstituierte Pyrenverbindung gemäß einem der Ansprüche 16 bis 17, die als Leuchtstoff in einem organischen EL Element eingesetzt wird.

19. Organische EL Anzeige mit einem organischen EL Element gemäß einem der Ansprüche 1 bis 15.

20. Organische EL Anzeige gemäß Anspruch 19, wobei die organische Anzeige entweder aus einem passiven Matrixfeld oder einem aktiven Matrixfeld besteht.

## Revendications

1. Elément EL organique comprenant :
une couche de mince film organique entre une électrode positive et une électrode négative, la couche de mince film organique contenant un composé de pyrène 1,3,6,8-tétrasubstitué représenté par la formule (1) suivante comme matériau luminescent : 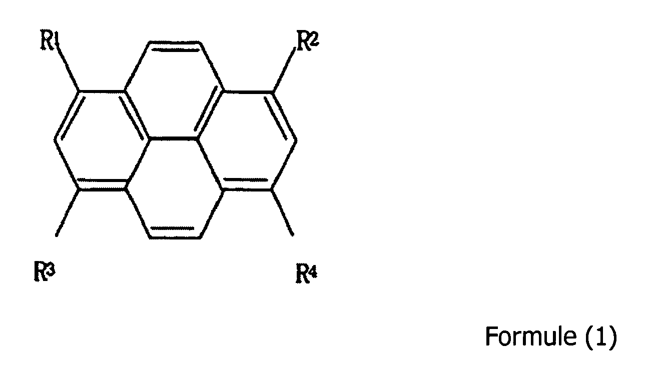 dans laquelle R¹ à R⁴ peuvent être identiques ou différents, et représentent un groupe représenté par la formule (2) suivante : 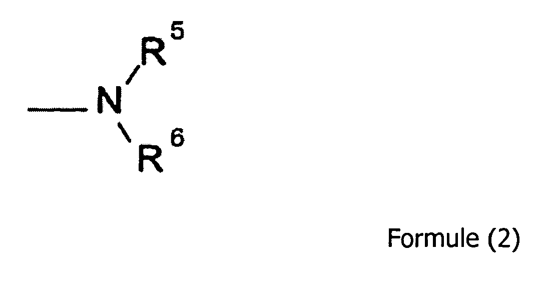 dans laquelle R⁵ et R⁶ peuvent être identiques ou différents, et représentent un groupe aryle dans lequel R⁵ et R⁶ ne forment pas un cycle par liaison.

2. Elément EL organique selon la revendication 1, dans lequel R¹ à R⁴ sont des groupes représentés par la formule (3) suivante, et le composé de pyrène 1,3,6,8-tétrasubstitué est le 1,3,6,8-tétrakis(N,N'-diphénylamino)pyrène : 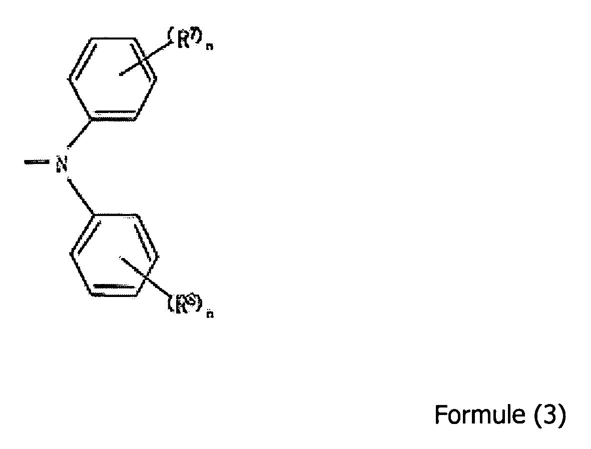 dans laquelle R⁷ et R⁸ peuvent être identiques ou différents, et représentent chacun au moins un atome d'hydrogène, au moins un groupe alkyle, ou au moins un groupe aryle ; et la lettre "n" représente un nombre entier égal à 1 ou plus.

3. Elément EL organique selon la revendication 1, dans lequel R¹ à R⁴ sont des groupes représentés par la formule (4) suivante, et le composé de pyrène 1,3,6,8-tétrasubstitué est le 1,3,6,8-tétrakis[N-(1-naphtyl)-N-phénylamino]pyrène : 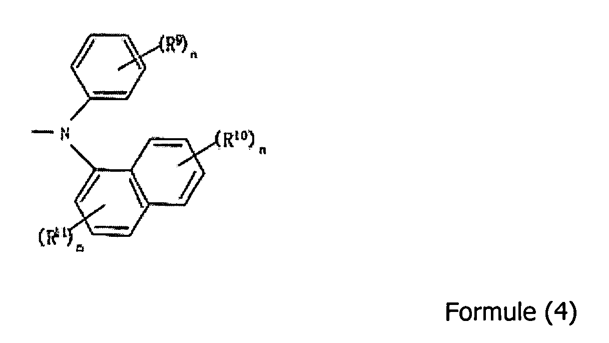 dans laquelle R⁹, R¹⁰ et R¹¹ peuvent être identiques ou différents, et représentent chacun au moins un atome d'hydrogène, au moins un groupe alkyle, ou au moins un groupe aryle ; et la lettre "n" représente un nombre entier égal à 1 ou plus.

4. Elément EL organique selon la revendication 1, dans lequel R¹ à R⁴ sont des groupes représentés par la formule (5) suivante, et le composé de pyrène 1,3,6,8-tétrasubstitué est le 1,3,6,8-tétrakis[4,4'-bis(α,α-diméthylbenzyl)diphénylamino]pyrène : 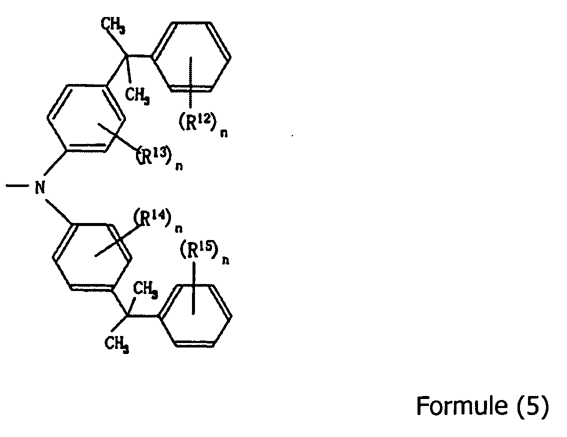 dans laquelle R¹², R¹³, R¹⁴ et R¹⁵ peuvent être identiques ou différents, et représentent chacun au moins un atome d'hydrogène, au moins un groupe alkyle, ou au moins un groupe aryle ; et la lettre "n" représente un nombre entier égal à 1 ou plus.

5. Elément EL organique selon l'une quelconque des revendications 1 à 4, dans lequel la couche de mince film organique comprend une couche émettant de la lumière et de transport d'électrons contenant le composé de pyrène 1,3,6,8-tétrasubstitué comme matériau luminescent.

6. Elément EL organique selon l'une quelconque des revendications 1 à 4, dans lequel la couche de mince film organique comprend une couche émettant de la lumière entre une couche de transport de trous positifs et une couche de transport d'électrons, la couche émettant de la lumière contenant le composé de pyrène 1,3,6,8-tétrasubstitué comme matériau luminescent.

7. Elément EL organique selon la revendication 6, dans lequel la couche émettant de la lumière est constituée du composé de pyrène 1,3,6,8-tétrasubstitué représenté par la formule (1).

8. Elément EL organique selon l'une quelconque des revendications 5 à 7, dans lequel la couche émettant de la lumière comprend un dérivé d'amine aromatique représenté par la formule (6) suivante : 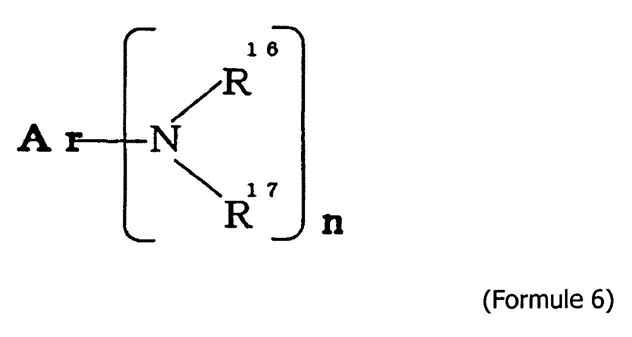 dans laquelle "n" est égal à 2 ou à 3, "Ar" représente un groupe choisi dans le groupe constitué d'un groupe aromatique divalent, d'un groupe aromatique trivalent, d'un groupe aromatique hétérocyclique divalent et d'un groupe aromatique hétérocyclique trivalent, et R¹⁶ et R¹⁷ peuvent être identiques ou différents représentant un parmi un groupe aromatique monovalent et un groupe aromatique hétérocyclique.

9. Elément EL organique selon la revendication 8, dans lequel le dérivé d'amine aromatique est choisi parmi la N,N'-dinaphtyl-N,N'-diphényl[1,1'-biphényl]-4,4'-diamine (NPD) représentée par la formule (7) suivante et un dérivé de celle-ci :

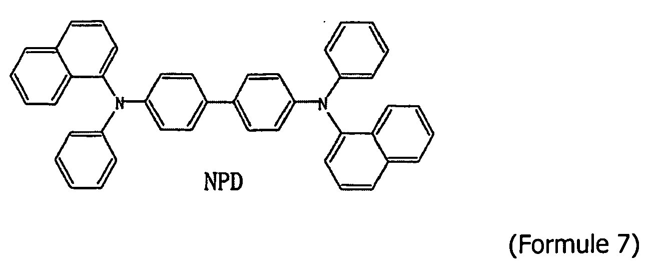

10. Elément EL organique selon l'une quelconque des revendications 5 à 9, dans lequel la couche émettant de la lumière comprend un dérivé de carbazole représenté par la formule (8) suivante : 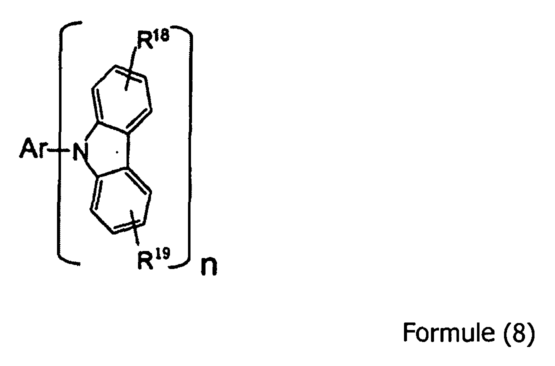 dans laquelle "Ar" représente un groupe choisi dans le groupe constitué par un groupe divalent ayant un cycle aromatique, un groupe trivalent ayant un groupe aromatique, un groupe divalent ayant un cycle aromatique hétérocyclique, et un groupe trivalent ayant un cycle hétérocyclique ; R¹⁸ et R¹⁹ représentent chacun indépendamment un atome d'hydrogène, un atome d'halogène, un groupe alkyle substitué ou non substitué, un groupe aralkyle substitué ou non substitué, un groupe alcényle substitué ou non substitué, un groupe aryle substitué ou non substitué, un groupe cyano substitué ou non substitué, un groupe amino substitué ou non substitué, un groupe acyle substitué ou non substitué, un groupe alcoxycarbonyle substitué ou non substitué, un groupe carboxyle substitué ou non substitué, un groupe alcoxy substitué ou non substitué, un groupe alkylsulfonyle substitué ou non substitué, un groupe hydroxyle substitué ou non substitué, un groupe amide substitué ou non substitué, un groupe aryloxy substitué ou non substitué, un cycle hydrocarboné aromatique substitué ou non substitué, ou un groupe hétérocyclique aromatique substitué ou non substitué ; et "n" est un nombre entier égal à 2 ou à 3.

11. Elément EL organique selon la revendication 10, dans lequel le dérivé de carbazole est choisi parmi le 4,4'-bis(9-carbazolyl)-biphényle (CBP) représenté par la formule (9) suivante et un dérivé de celui-ci :

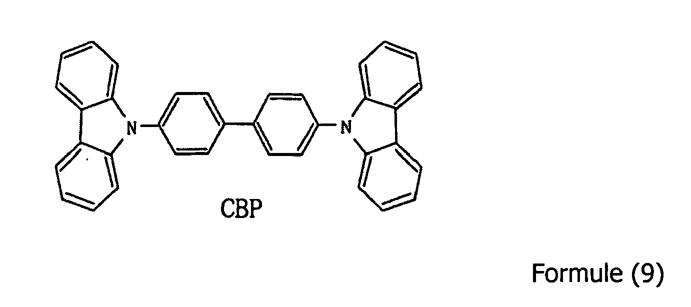

12. Elément EL organique selon l'une quelconque des revendications 5 à 11, dans lequel la couche émettant de la lumière comprend un complexe d'oxine représenté par la formule (10) suivante : 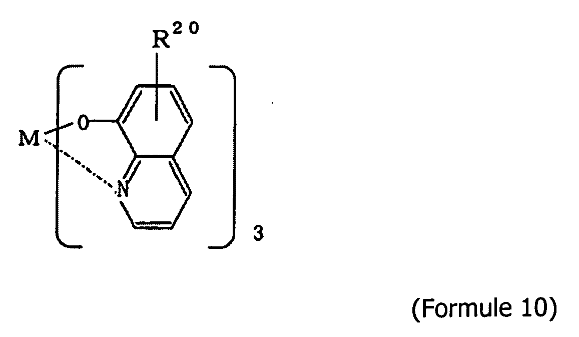 dans laquelle "M" représente un atome de métal, et R²⁰ représente un atome d'hydrogène, un atome d'halogène, un groupe alkyle, un groupe aralkyle, un groupe alcényle, un groupe aryle, un groupe cyano, un groupe amino, un groupe acyle, un groupe alcoxycarbonyle, un groupe carboxyle, un groupe alcoxy, un groupe alkylsulfonyle, un groupe hydroxyle, un groupe amide, un groupe aryloxy, un cycle hydrocarboné aromatique, ou un groupe hétérocyclique aromatique. Ceux-ci peuvent de plus être substitués par des substituants.

13. Elément EL organique selon la revendication 12, dans lequel le complexe d'oxine est un complexe d'aluminium quinoléine (Alq) représenté par la formule (11) suivante :

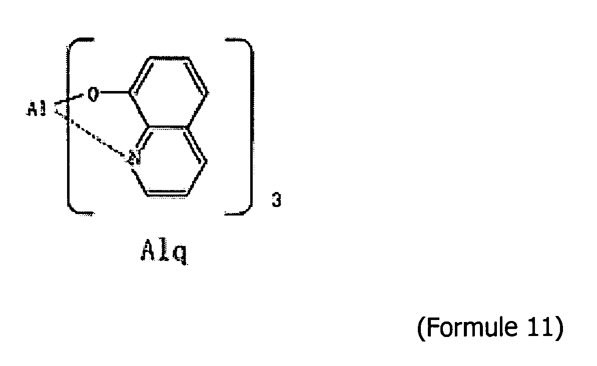

14. Elément EL organique selon l'une quelconque des revendications 5 à 13, dans lequel la couche de transport d'électrons comprend la 2,9-diméthyl-4,7-diphényl-1,10-phénanthroline (BCP) représentée par la formule (12) suivante comme matériau de transport d'électrons :

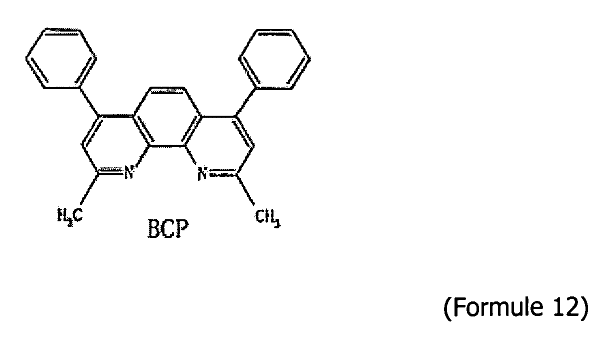

15. Elément EL organique selon l'une quelconque des revendications 1 à 14, dans lequel l'élément EL organique émet de la lumière verte.

16. Composé de pyrène 1,3,6,8-tétrasubstitué représenté par la formule (1) suivante 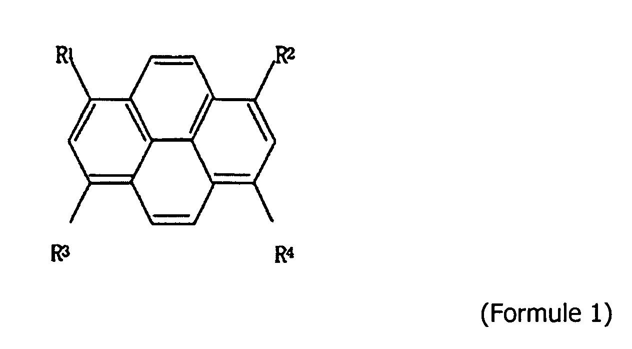 dans laquelle R¹ à R⁴ sont des groupes représentés par la formule (4) suivante, et le composé de pyrène 1,3,6,8-tétrasubstitué est le 1,3,6,8-tétrakis[N-(1-naphytyl)-N-phénylamino]pyrène : 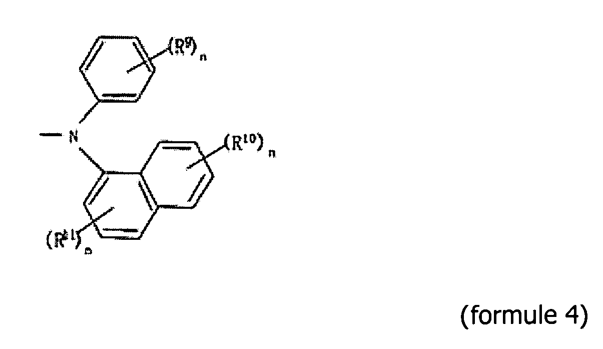 dans laquelle R⁹, R¹⁰ et R¹¹ peuvent être identiques ou différents, et représentent chacun au moins un atome d'hydrogène, au moins un groupe alkyle, ou au moins un groupe aryle ; et la lettre "n" représente un nombre entier égal à 1 ou plus.

17. Composé de pyrène 1,3,6,8-tétrasubstitué représenté par la formule (1) suivante 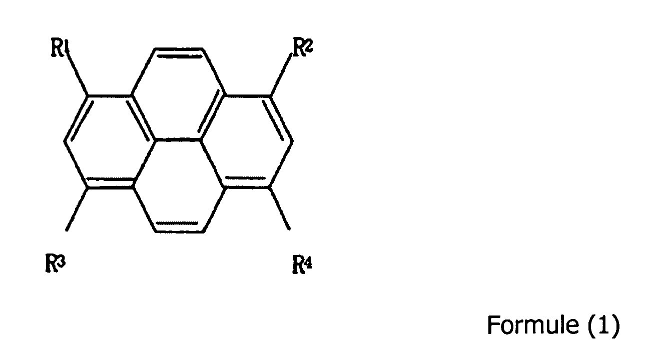 dans laquelle R¹ à R⁴ sont des groupes représentés par la formule (5) suivante, et le composé de pyrène 1,3,6,8-tétrasubstitué est le 1,3,6,8-tétrakis[4,4'-bis(α,α-diméthylbenzyl)diphénylamino]pyrène : 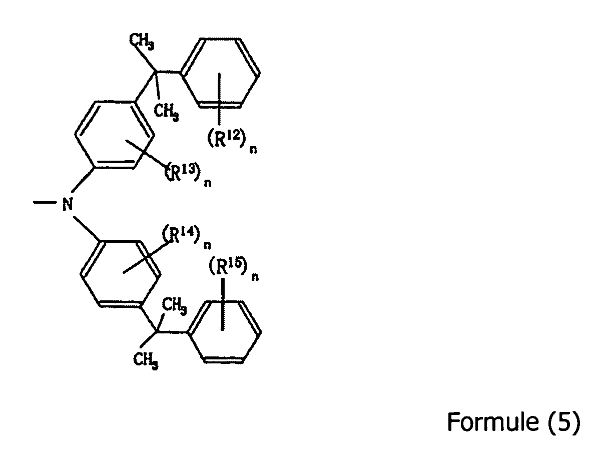 dans laquelle R¹², R¹³, R¹⁴ et R¹⁵ peuvent être identiques ou différents, et représentent chacun au moins un atome d'hydrogène, au moins un groupe alkyle, ou au moins un groupe aryle ; et la lettre "n" représente un nombre entier égal à 1 ou plus.

18. Composé de pyrène 1,3,6,8-tétrasubstitué selon l'une quelconque des revendications 16 à 17 utilisé comme matériau luminescent dans un élément EL organique.

19. Affichage EL organique, comprenant un élément EL organique selon l'une quelconque des revendications 1 à 15.

20. Affichage EL organique selon la revendication 19, dans lequel l'affichage organique est un parmi un panneau à matrice passive et un panneau à matrice active.
